(19) Europäisches Patentamt
European Patent Office
Office européen des brevets

(11) EP 1 739 095 A1

(12) # EUROPEAN PATENT APPLICATION
published in accordance with Art. 158(3) EPC

(43) Date of publication:
**03.01.2007 Bulletin 2007/01**

(21) Application number: **05704132.9**

(22) Date of filing: **26.01.2005**

(51) Int Cl.:
***C08B 37/00*** *(2006.01)*

(86) International application number:
**PCT/JP2005/000995**

(87) International publication number:
**WO 2005/073255 (11.08.2005 Gazette 2005/32)**

(84) Designated Contracting States:
**AT BE BG CH CY CZ DE DK EE ES FI FR GB GR HU IE IS IT LI LT LU MC NL PL PT RO SE SI SK TR**

(30) Priority: **30.01.2004 JP 2004024894**

(71) Applicant: **TOHO CHEMICAL INDUSTRY CO., LTD.**
**Tokyo 104-0044 (JP)**

(72) Inventors:
- **TAKEDA, Hiromitsu,**
**c/o Toho Chem. Ind. Co., Ltd.**
**Sodegaura-shi,**
**Chiba 299-0296 (JP)**
- **MORI, Yoshihiko,**
**c/o Toho Chem. Ind. Co., Ltd.**
**Sodegaura-shi,**
**Chiba 299-0296 (JP)**
- **UEDA, Hiromichi,**
**c/o Toho Chem. Ind. Co., Ltd.**
**Sodegaura-shi,**
**Chiba 299-0296 (JP)**

(74) Representative: **Schaad, Balass, Menzl & Partner AG**
**Dufourstrasse 101**
**Postfach**
**8034 Zürich (CH)**

(54) **CATION-MODIFIED PURIFIED GALACTOMANNAN POLYSACCHARIDE AND COSMETIC COMPOSITION CONTAINING THE SUBSTANCE**

(57) [Subject matter] The present invention presents a cationic polymer. When a hair treatment composition containing the cationic polymer is used to damaged hair, excellent conditioning effects are shown. When a skin cosmetic treatment composition containing the cationic polymer, foam formation and foam quality are improved and good use feeling is obtained.

[Means to solve problem] The present inventions relates to cation-modified purified galactomannan polysaccharides prepared by purifiying a crude galactomannan polysaccharide obtained from albumens of seeds which comprises a main chain composed of mannose units and a side chain composed of galactose units and has mannose to galactose ratio of 4:1 and/or 3:1 to produce purified galactomannan polysaccharide in which the galactomannan content is 80 wt%, and partially substituting hydroxyl groups of the purified galactomannan polysaccharide with a quaternary nitrogen-containing group of Formula (1), wherein a cation charge density derived from the quaternary nitrogen-containing group can be obtained in a specific range, and cosmetic composition containing the same.

Chemical formula (1)

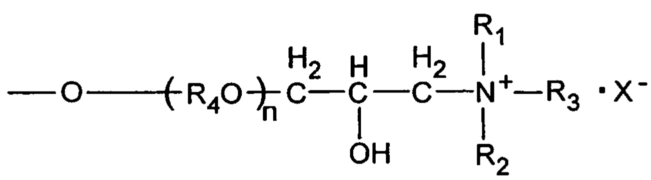

(where $R_1$ and $R_2$ each independently represent an alkyl group having 1 to 3 carbon atoms; $R_3$ represents an alkyl group having 1 to 24 carbon atoms; X-represents an anion; n is either 0 or 1 to 30; and, when n is 1 to 30, $(R_4O)_n$ represents a polymer residue of an alkylene oxide having 2 to 4 carbon atoms and forms a polyalkylene glycol chain composed of a single alkylene oxide and/or a polyalkylene glycol chain composed of two or more types of alkylene oxide).

EP 1 739 095 A1

## Description

TECHNICAL FIELD

The present invention relates to a specific cation-modified galactomannan polysaccharide functioning as a cationic polymer which, when contained in a cosmetic composition, improves adhesion to hair and skin, exerts good conditioning effects, and achieves satisfactory finish free of rigidity after drying, and to a cosmetic composition, in particular, a hair treatment composition, containing the cation-modified galactomannan polysaccharide.

BACKGROUND TECHNOLOGY

A hair treatment composition for hair cleansing contains a conditioning agent for reducing damage on tangled hair during washing and rinsing and for improving the feeling of hair after washing. Substances that yield conditioning effects must adsorb onto hairs; thus, cationic polymers having ionic adsorption ability are typically used as such substances. Among these polymers, cation-modified cellulose derivatives, cation-modified guar gum, and dimethyldiallyl ammonium chloride derivatives have been widely used. For example, Patent Document discloses the use of a cation-modified cellulose derivative, which incorporates a quaternary nitrogen-containing group, in a shampoo or hair treatment composition. Patent Document 2 discloses cation-modified guar gum having a ratio of mannose (galactomannan polysaccharide) constituting the main chain to galactose constituting the side chain of 2:1, the cation modified guar gum being used as a cationic conditioning polymer in hair care products, such as shampoos, hair rinses, etc., together with insoluble, nonvolatile silicone to improve the appearance of hair. Patent Document 3 discloses a cation-modified galactomannan polysaccharide prepared by depolymerizing a galactomannan polysaccharide, i.e., guar gum or locust bean gum, by decomposition with an enzyme or the like, followed by cationic modification. Patent Document 4 discloses the use of a cation-modified galactomannan polysaccharide prepared by modifying a galactomannan polysaccharide, i.e., guar gum or locust bean gum, with a hydroxyalkyl group, followed by cation modification and depolymerization, in a hair treatment composition and a skin care composition.

However, these cation-modified polymers cause rigidity that is unsatisfactory for users after drying and insufficient conditioning effects during rinsing due to the ratio of the main chain to the side chain of the galactomannan polysaccharide, structural differences caused by the presence or absence of hydroxyalkyl groups, and the difference in viscosity caused depolymerization. In order to overcome these problems, cation-modified locust beam gum and cation-modified tara gum are used. Patent Document 5 discloses use of cation-modified locust beam gum in a hair care product, such as a shampoo or rinse, etc. and reports improved conditioning effects during rinsing and less frictional feel and rigidity after drying in comparison with a product with a cation-modified guar gum. Non-patent Document1 discloses an example of a hair care product, such as a shampoo or conditioner, containing this cation-modified locust beam gum. Patent Document 6 discloses cation-modified tara gum is suitable as a component of a hair care product, such as a shampoo or rinse, or a skin care composition, such as body cleansing preparation, because it improves the foam quality and feeling.

Patent Document 1: Japanese Patent Publication No. Sho 47-20635 (p. 5)
Patent Document 2: Japanese Patent Application Laid-open No. Hei 4-364111 (pp. 1-6)
Patent Document 3: Japanese Patent Application Laid-open No. Hei 7-173029 (pp. 1-7)
Patent Document 4: Japanese Patent No. 3349219 (pp. 1-6)
Patent Document 5: Japanese Patent Application Laid-open No. 2000-103724(pp. 1 to 6)
Patent Document 6: Japanese Patent Application Laid-open No. 2003-327603 (pp. 1-23)
Non-patent Document 1: FRAGRANCE JOURNAL 2003-10 (p. 34)

DISCLOSURE OF THE INVENTION

PROBLEM TO BE SOLVED BY THE INVENTION

Diversified fashion trends have increased the frequency of using hair colors and hair dyes, which significantly damage hair and require conditioning agents to achieve greater conditioning effects, in particular, to render smoothness to hair during the use. For the purpose of improving lack of conditioning effects at use and worse of use feeling which were caused by cation-modified cellulose derivatives, depolymerized cation-modified galactomannan polysaccharides, cation-modified guar gum, and the like, cation-modified locust bean gum and cation-modified tara gum have been invented by, respectively, cation-modifying locust gum and tara gum. However, when they are used to treat damaged hair, hair becomes tangled at the fingers during the rinsing and the finish after drying is rarely satisfactory. Moreover, when they are incorporated in skin care compositions, satisfaction of users (use feeling) is low and appearance is poor

due to the presence of water-insoluble substances.

MEANS TO SOLVE THE INVENTION

Under these circumstances, the inventors have conducted extensive investigations to search for a compound that can render improved smoothness to wet hair and improved finish after drying when the compound is contained in a hair treatment composition composed of the conventional cation-modified locust bean gum or a cation-modified tara gum and that can improve the foam quality and the feeling of skin after use when the compound is contained in a skin care composition. As a result, the inventors have found that a particular cation-modified galactomannan polysaccharide has excellent conditioning effects, improves the smoothness of wet hair, enhances foam formation (lathering) and foam quality, and ensures the improvement of feeling when the cation-modified purified galactomannan polysaccharide is contained in a cosmetic composition. This cation-modified purified galactomannan polysaccharide is prepared by introducing a particular amount of a quaternary nitrogen-containing group into a galactomannan polysaccharide (hereinafter also referred to as "purified galactomannan polysaccharide") which is obtained by purifying crude galactomannan polysaccharide, comprises a main chain composed of mannose units and a side chain composed of galactose units, and has a mannose to galactose ratio of 4:1 and/or 3:1 and contains 80 wt% or more of galactomannan, and aqueous solution of which can form a gel with an aqueous solution of xanthan gum and/or carrageenan. The cation charge density of this cation-modified galactomannan polysaccharide is within a particular range.

MEANS TO SOLVE THE PROBLEM

Thus, the present invention relates to a cation-modified purified galactomannan polysaccharide prepared by partially substituting hydroxyl groups of a purified galactomannan polysaccharide with a quaternary nitrogen-containing group of Formula (1), the purified galactomannan polysaccharide being prepared by purifyng a crude galactomannan polysaccharide, comprising a main chain composed of mannose units and a side chain composed of galactose units, and containing 80 wt% or more of galactomannan:

Chemical formula (1)

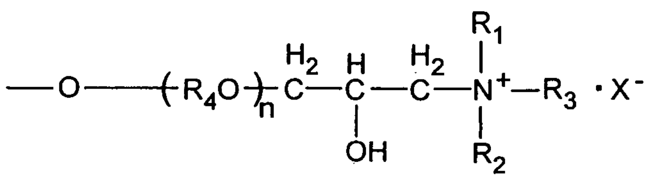

(1)

(where $R_1$ and $R_2$ each independently represent an alkyl group having 1 to 3 carbon atoms; $R_3$ represents an alkyl group having 1 to 24 carbon atoms; X-represents an anion; n is either 0 or 1 to 30; and, when n is 1 to 30, $(R_4O)_n$ represents a polymer residue of an alkylene oxide having 2 to 4 carbon atoms and forms a polyalkylene glycol chain composed of a single alkylene oxide and/or a polyalkylene glycol chain composed of two or more types of alkylene oxide), wherein the cation charge density derived from the quaternary nitrogen-containing group is in the range of 0.1 to 3.0 meq/g. An aqueous solution of the purified galactomannan polysaccharide can form a gel with an aqueous solution of xanthan gum and/or carrageenan. The purified galactomannan polysaccharide has a mannose to galactose ratio of 4:1 and/or 3:1.

The inventive cation-modified purified galactomannan polysaccharide reduces water-insoluble matters in crude galactomannan polysaccharides, so that when the inventive cation-modified purified galactomannan polysaccharide is contained in a hair treatment composition, such as a shampoo or rinse, it can achieve conditioning effects superior to those achieved by hair treatment compositions containing the conventional cation-modified locust bean gum (hereinafter also referred to as "nonpurified cation-modified locust bean gum") and cation-modified tara gum (hereinafter referred to as "nonpurified cation-modified tara gum") prepared by cation-modifying locust bean gum and tara gum containing less than 80 wt% of galactomannan. Moreover, the hair treatment composition containing the inventive cation-modified purified galactomannan polysaccharide will yield excellent feeling after drying. When the inventive cation-modified purified galactomannan polysaccharide is contained in a skin care composition, foam formation and foam quality can be improved and good finish feel can be achieved. Thus, the present invention can provide a cosmetic composition that has excellent use feeling compared to conventional products.

One of galactomannan polysaccharides used in the present invention contains a main chain composed of mannose units and a side chain composed of galactose units and is a nonionic polysaccharide having a mannose to galactose ratio of 4:1. The galactomannan polysaccharide is a natural water-soluble gum obtained from albumens of seeds of locust bean (*Ceratonia Siliqua*), a perennial leguminous plant grown in Mediterranean regions and is commonly called "locust bean gum" or "carob gum". The present invention uses a purified galactomannan polysaccharide prepared by purifying a crude galactomannan polysaccharide obtained from albumens of seeds of locust bean so that the galactomannan content is 80 wt% or more and preferably 83 wt% or more.

Another galactomannan polysaccharide used in the present invention contains a main chain composed of mannose units and a side chain composed of galactose units and is a nonionic polysaccharide having a mannose to galactose ratio of 3:1. This galactomannan polysaccharide is a natural water-soluble gum obtained from albumens of seeds of tara *(Caesalpinia Spinosa),* a leguminous plant native of the South American Andes, and is commonly called "tara gum". The present invention uses a purified galactomannan polysaccharide prepared by purifying a crude galactomannan polysaccharide obtained from albumens of seeds of tara so that the galactomannan content is 80 wt% or more and preferably 83 wt% or more.

An example process for purifying crude galactomannan polysaccharide to increase the galactomannan content to 80 wt% or more includes dissolving crude galactomannan polysaccharide (hereinafter also referred to as "nonpurified galactomannan polysaccharide") containing less than 80 wt% of galactomannan in water to prepare an aqueous solution, filtering the aqueous solution to remove water-insoluble matters, precipitating the filtrate in an organic solvent, and drying the resulting precipitates. Alternatively, the purified galactomannan polysaccharide may be obtained by removing the shell portions of the seeds and milling only the cores of albumens left thereby. Although the crude galactomannan polysaccharide is purified by such conventional processes, the purifying process is not limited to these.

It is well known that locust bean gum having a mannose to galactose ratio of 4:1 and tara gum having a mannose to galactose ratio of 3:1 have different properties, such as rheological properties, from those of guar gum having a mannose to galactose ratio of 2:1.

A cation-modified purified galactomannan polysaccharide of the present invention can be produced by reacting a galactomannan polysaccharide with a glycidyltrialkyl ammonium salt having a quaternary nitrogen-containing group or a 3-halogeno-2-hydroxypropyltrialkyl ammonium salt having a quaternary nitrogen-containing group, the galactomannan polysaccharide being prepared by purifying a crude galactomannan polysaccharide and containing 80 wt% or more of galactomannan and having a mannose to galactose ratio of 4:1 (in such a case, the polysaccharide is referred to as "purified locust bean gum") or a mannose to galactose ratio of 3:1 (in such a case, the polysaccharide is referred as "purified tara gum"). The galactomannan polysaccharide may be a mixture of the two gums. The reaction is conducted in a suitable solvent, preferably a hydrous alcohol, in the presence of an alkali. Such an introduction of a quaternary nitrogen-containing group can be conducted by known techniques, but the introduction process is not particularly limited. For example, the reaction between the galactomannan polysaccharide and the quaternary nitrogen-containing group of the glycidyltrialkyl ammonium salt or the 3-halogeno-2-hydroxypropyltrialkyl ammonium salt may be conducted after alkylene oxide having 2 to 4 carbon atoms are added to part of hydroxyl groups contained in a purified galactomannan polysaccharide having a mannose to galactose ratio of 4:1, thereby yielding the cation-modified purified galactomannan polysaccharide of the present invention (hereinafter, the cation-modified galactomannan polysaccharide having a mannose to galactose ratio of 4:1 is also referred to as "purified cation-modified locust bean gum). Moreover, in order to prevent aggregation of the purified locust bean gum in the solvent during the reaction, an inorganic salt, preferably sodium chloride, may be added to the reaction system. Moreover, in order to prevent aggregation of the purified locust bean gum, to enhance the dispersibility of the solute and to increase the rate of reaction, an alkali or inorganic salt may be dissolved or dispersed in the reaction solvent, and subsequently the purified locust bean gum may be added and dissolved or dispersed to introduce the quaternary nitrogen-containing group.

A galactomannan polysaccharide having a mannose to galactose ratio of 3:1 and containing 80 wt% or more of galactomannan (hereinafter also referred to as "purified tara gum") can be modified with cation by the same process as that for producing the purified locust bean gum described above to produce an inventive cation-modified purified galactomannan polysaccharide (hereinafter, the inventive cation-modified galactomannan polysaccharide having a mannose to galactose ratio of 3:1 is also referred to as "purified cation-modified tara gum").

Alternatively, a cation-modified purified galactomannan polysaccharide containing 80 wt% more of galactomannan can be produced by removing water-insoluble matters, such as shells, by milling, classifying, or filtering a crude galactomannan polysaccharide containing less than 80 wt% of galactomannan after the crude galactomannan polysaccharide was cation-modified by a similar process of the above-described process.

Alternatively, an inventive cation-modified purified galactomannan polysaccharide can be produced by cation-modifying a mixture of a purified locust bean gum and a purified tara gum by the same process employed for the purified locust bean gum.

In the present invention, a quaternary nitrogen-containing group represented by general formula (1) is introduced to a purified locust bean gum, i.e., a galactomannan polysaccharide having a mannose to galactose ratio of 4:1, or a

purified tara gum, i.e., a galactomannan polysaccharide having a mannose to galactose ratio of 3:1. Examples of $R_1$ and $R_2$ in the quaternary nitrogen-containing group include methyl, ethyl and propyl. Examples of $R_3$, which is an alkyl group having 1 to 24 carbon atoms, include methyl, ethyl, propyl, octyl, decyl, dodecyl, tetradecyl, hexadecyl, octadecyl and dococyl. Examples of $R_4O$ include ethoxy, propoxy and butoxy. Examples of anion X- include halogen ions such as a chlorine ion, a bromine ion and an iodine ion, a methyl sulfate ion, an ethyl sulfate ion and an acetate ion.

By cation-modifying a purified galactomannan polysaccharide containing 80 wt% or more of galactomannan, the inventive cation-modified purified galactomannan polysaccharide prepared exhibits adsorption to hair and skin, provides satisfactory smoothness, and is thus suitable as a component of cosmetic compositions. For example, when the inventive cation-modified purified galactomannan polysaccharide is contained in a hair treatment composition, finger-passing during hair-rinsing can be reduced and the finish feel after drying can be improved. If the inventive polysaccharide is contained in a skin care composition, e.g., a body cleansing preparation, the foam quality can be improved, and the feel of the skin to the feeling after use can be improved. Thus, the present invention is also directed to these cosmetic compositions containing the inventive cation-modified purified galactomannan polysaccharide.

The cation charge density derived from the quaternary nitrogen-containing group in the inventive cation-modified purified galactomannan polysaccharide is in the range of 0.1 to 3.0 meq/g, and preferably 0.5 to 2.5 meq/g. At a cation charge density of less than 0.1 meq/g, the adsorption of the cation-modified purified galactomannan polysaccharide to hair and skin will be insufficient; thus, a hair treatment composition or skin care composition, such as a shampoo, a conditioner, or a body cleansing preparation, containing this cation-modified purified galactomannan polysaccharide will exhibit no improvement. A hair treatment composition or a skin care composition containing a purified cation-modified galactomannan polysaccharide having a charge density exceeding 3.0 meq/g will obstruct formation of foam and give stickiness and sliminess feel disagreeable for users during the use. Moreover, skin or hair after the treatment will be undesirably rigid and sticky.

Here, the "cation charge density" derived from the quaternary nitrogen-containing group in the cation-modified purified galactomannan polysaccharide refers to the equivalent weight of the quaternary nitrogen-containing group of Formula (1) per gram of the cation-modified purified galactomannan polysaccharide. In general, the nitrogen content derived from the quaternary nitrogen-containing group is determined by Kjeldahl analysis (The Japanese Standards of Cosmetic Ingredients, old edition, General Test Methods, Nitrogen Determination, Method 2), and the equivalent weight can be calculated from the observed nitrogen content. However, the purified galactomannan polysaccharide having a mannose to galactose ratio of 4:1 or 3:1 used in the present invention contains nitrogen. The nitrogen content derived from the purified galactomannan polysaccharide must be subtracted from the observed nitrogen content of the inventive cation-modified purified galactomannan polysaccharide to determine the nitrogen content derived from the quaternary nitrogen-containing group. For example, suppose an inventive cation-modified purified galactomannan polysaccharide, in which the quaternary nitrogen-containing group represented by formula (1) contains $R_1$, $R_2$, and $R_3$ each representing methyl, X- representing chlorine ion, and n being zero, is prepared by introducing a cation to a purified locust bean gum, and the nitrogen content of that inventive polysaccharide is determined to be 2.16% by Kjeldahl analysis. In such a case, the cation charge density can be determined by the following equation (the purified locust bean gum used in the present invention usually contains about 0.90% of nitrogen):

<u>Equation 1</u>

$$\text{cation charge density (meq/g)} = (\text{nitrogen content (\%) derived from quaternary nitrogen-containing group} \times 1000)/(\text{atomic weight of nitrogen (14.0)} \times 100)$$

$$= (2.16 - 0.90)/1.40 = 0.90$$

The "galactomannan content" in the crude galactomannan polysaccharide refers to the mass percent of galactomannan contained per gram of a crude galactomannan polysaccharide, i.e., a natural water-soluble gum from which the inventive cation-modified purified galactomannan polysaccharide is made, based on solid content. Although an example method of determining the galactomannan content by using absorbance after enzymic decomposition is given below, the method is not limited to this and may be a liquid chromatographic method, a thin-layer chromatographic method, or the like. A sample of about 20 mg is accurately weighted, placed in a centrifugal tube, and mixed with 5 mL of ethanol. The mixture is then incubated at 85°C to 95°C and separated by centrifugation into an upper phase and a lower phase. To the lower phase, 10.0 mL of a 50 mM acetic acid buffer solution (pH 4.5) is added, and the resulting mixture is heated and left to stand at 40°C for 12 hours. Subsequently, 20 μL of β -mannanase is added to the mixture, and the resulting mixture is cultured at 40°C for 180 minutes. The mixture is then centrifuged and 0.1 mL of supernatant

is sampled. The supernatant is mixed with 0.2 mL of a 50 mM acetic acid buffer solution (pH 4.5) and 20 μ L of α -galactosidase. The resulting solution is incubated at 40°C and mixed with 2.5 mL of a 200 mM tris buffer solution and then with 0.1 mL of β -nicotineamide adeninedinucleotide (0.1 g/10 mL) and 8 μL of galactose dehydrogenase. The resulting mixture is incubated at 40°C for 60 minutes. Subsequently, the absorbance of the solution at 340 nm is measured. The galactose content is then determined by the following equation from the observed absorbance (the galactomannan content is determined from the galactose content using the mannose to galactose ratio of the sample):

Equation 2

$$\text{galactose content (\%)} = (E - E_0) \times (1/W) \times A^*$$

E: absorbance at 340 nm (sample solution)
$E_0$: absorbance at 340 nm (blank)
A*: conversion factor for determining galactose content
W: sample-collecting amount (mg)

To be more specific, using a galactomannan assay kit produced by Biocon (Japan) Ltd., the galactomannan content in the purified locust bean gum used in the present invention is determined. Given that the conversion factor A* determined from the calibration curve is 1384.6, that the absorbance of the blank at 340 nm is 0.276, that 17.8 mg of the purified locust bean gum (based on solid content) is sampled, and that the absorbance of the sample solution at 340 nm is 0.502, the calculated galactose content is 17.6%. Since the locust bean gum has a mannose to galactose ratio of 4:1, the galactomannan content is 88.0%.

The inventive purified cation-modified galactomannan polysaccharide is particularly suitable as a component of a cosmetic composition. For example, a hair care composition containing the inventive cation-modified purified galactomannan polysaccharide will render improved smoothness to wet hair, achieve improved conditioning effect, and ensure a more satisfactory finish feel as compared with a hair treatment composition containing the conventional nonpurified cation-modified locust bean gum or the conventional nonpurified cation-modified tara gum prepared by cation-modifying an nonpurified locust gum or nonpurified tara gum containing less than 80 wt% of galactomannan. Because nonpurified cation-modified locust bean gum and nonpurified tara gum contain water-insoluble matters in addition to galactomannan polysaccharide which is a natural water-soluble gum, skin care compositions containing the conventional gums do not have satisfactory feeling due to the presence of water-insoluble matters. In contrast, skin care compositions containing inventive cation-modified purified galactomannan polysaccharide will not cause any lowering of feeling due to fewer water-insoluble matters by the purification and will produce satisfactory foaming. Thus, the present invention can provide a high quality skin care composition.

When the cation-modified purified galactomannan polysaccharide of the present invention is contained in a hair treatment composition or a skin care composition, the content thereof is preferably 0.05 to 5 wt% relative to 100 wt% of the entire composition. At a content less than 0.5 percent by mass, its conditioning effects tend to be insufficient. At a content exceeding 5 wt%, the composition will give the user sliminess and stickiness feeling and degrade the smoothness, thereby causing the use feeling worse.

To a hair treatment composition of the present invention, a cationic water-soluble polymer or amphoteric water-soluble polymer may be added, in addition to a cation-modified galactomannan polysaccharide, in order to further enhance the conditioning effect of the composition. However, the amount of the additional polymer added should be adjusted so as not to interfere with the emulsifiability and soft texture to hair of a cation-modified galactomannan polysaccharide used in combination. The amount in question added is preferably 5% by weight or lower, with respect to the weight (100% by weight) of the entire composition. If the amount added exceeded the above range, the membrane-forming activity and hair-setting performance of a cation-modified galactomannan polysaccharide used in combination would be impaired. Moreover, if the composition to which that additional polymer is added is for treating the skin, the skin would be so slimy as to displease the user.

Suitable cationic water-soluble polymers and amphoteric water-soluble polymers include the following compounds, but they are not necessarily limited to these compounds.

The cationic water-soluble polymer may include a quaternary nitrogen-based cationic polysaccharide (cation-modified hydroxyethyl cellulose, cation-modified guar gum, cation-modified locust-bean guar gum, cation-modified starch, cation-modified tara gum, cation-modified tamarind gum, etc.), dimethyldiallyl ammonium chloride derivatives (copolymers of dimethyldiallyl ammonium chloride and acrylamide, polychlorinated dimethylmethylenepiperidinium, etc.), vinylpyrrolidone derivatives (copolymers of vinylpyrrolidone and dimethylaminoethylmethacrylate, copolymers of vinylpyrrolidone and methacrylamidopropyltrimethylammonium chloride, copolymers of vinylpyrrolidone and methylvinylimidazolium chloride, etc.), and methacrylate derivatives (copolymers of methacryloylethyldimethylbetaine, methacryloylethyltrimethylammonium chloride and methacrylic acid 2-hydroxyethyl, copolymers of methacryloylethyldimethylbetaine, methacryloylethyltrimethylammonium chloride, and methacrylic acid methoxypolyethylene glycol, etc.).

The amphoteric water-soluble polymer may include amphoterized starch, dimethyldiallylammonium chloride derivatives (copolymers of acrylamide, acrylic acid and dimethyldiallylammonium chloride, copolymers of acrylic acid and dimethyldiallylammonium chloride, etc.), and methacrylate derivatives (copolymers of polymethacryloylethyldimethylbetaine, N-methacryloyloxyethyl N,N-dimethylammonium-α-methylcarboxybetaine and alkyl methacrylate, etc.)

A cosmetic preparation according to the present invention can be obtained by adding a cation-modified galactomannan polysaccharide of the present invention to a prescribed system at a specified amount by a commonly known method, and other components to be used in combination are not limited to specific ones, but any components generally used in such cosmetic preparations may be used. Such additives to be used in combination include followings.

The anionic surfactant may include C 8-24 alkyl sulfate, C 8-24 alkyl ether sulfate, C 8-24 alkyl benzene sulfonate, C 8-24 alkyl phosphate, C 8-24 polyoxyalkylenealkyl ether phosphate, C 8-24 alkyl sulfosuccinate, C 8-24 polyoxyalkylenealkyl ether sulfosuccinate, C 8-24 acyl alaninate, C 8-24 acyl N-methyl-β-alaninate, C 8-24 acyl glutamate, C 8-24 acyl isethionate, C 8-24 acyl sarcosinate, C 8-24 taurinate, C 8-24 acyl methyl taurinate, α-sulfofatty acid ester salt, ether carbonate, polyoxyalkylene fatty acid monoethanol amide sulfonate, C 8-24 long chain carboxylate, etc. (C 8-24 means 8 to 24 carbon atoms.)

The nonionic surfactant may include alkanol amide, glycerin fatty acid ester, polyoxyalkylenealkyl ether, polyoxyalkyleneglycol ether, polyoxyalkylenesorbitan fatty acid ester, sorbitan fatty acid ester, polyoxyalkylenesorbit fatty acid ester, sorbit fatty acid ester, polyoxyalkyleneglycerin fatty acid ester, polyoxyalkylene fatty acid ester, polyoxyalkylenealkylphenyl ether, tetrapolyoxyalkylene ethylenediamine-condensed substances, sucrose fatty acid ester, polyoxyalkylene fatty acid amide, polyoxyalkyleneglycol fatty acid ester, polyoxyalkylene castor oil derivatives, polyoxyalkylenehardened castor oil derivatives, alkylpolyglycoside, polyglycerin fatty acid ester, etc.

The amphoteric surfactant may include alkyl (8-24 carbons) amidopropylbetaine, alkyl (8-24 carbons) carboxybetaine, alkyl (8-24 carbons) sulfobetaine, alkyl (8-24 carbons) sulfobetaine, alkyl (8-24 carbons) hydroxysulfobetaine, alkyl (8-24 carbons) amidopropylhydroxysulfobetaine, alkyl (8-24 carbons) hydroxyphosphobetaine, alkyl (8-24 carbons) aminocarboxylate, alkyl (8-24 carbons) imidazoliumbetaine, alkyl (8-24 carbons) amineoxide, alkyl (8-24 carbons) phosphate esters containing a tertiary or quaternary nitrogen group, etc.

In addition to the cationic or nonionic water-soluble polymers described above, an anionic or nonionic polymer may be added to a hair treatment composition or skin care composition of the present invention in order to adjust the viscosity of the composition or improve the performance of the composition in maintaining the finished style, so long as the addition of the polymer does not interfere with the expected effects of the composition of the present invention.

Such anionic polymers may include acrylate derivatives (polyacrylic acid and its salts, and copolymers of acrylic acid, acryl amide and ethyl acrylate, and their salts), methacrylate derivatives (polymethacrylic acid and its salts, copolymers of methacrylic acid, acryl amide, diacetone acrylamide, alkylesteracrylate and alkylestermethacrylate, and their salts), crotonate derivatives (copolymers of vinylacetate and crotonic acid, etc.), maleate derivatives (copolymers of anhydrous maleic acid and diisobutylene, copolymers of isobutylene and maleic acid, etc.), polyglutamic acid and its salts, hyaluronic acid and its salts, carboxymethyl cellulose, carboxyvinyl polymer, etc.

The nonionic polymer may include acrylate derivatives (copolymers of hydroxyethyl acrylate and methoxyethyl acrylate, amide polyacrylate, etc.), vinylpyrrolidone derivatives (polyvinylpyrrolidone, copolymers of vinylpyrrolidone and vinyl acetate, etc.), polyoxyalkylene glycol derivatives (polyethylene glycol, polypropylene glycol, etc.), cellulose derivatives (methyl cellulose, hydroxyethyl cellulose, hydroxypropyl cellulose, etc.), polysaccharides and their derivatives (guar gum, locust-bean gum, dextran, etc.), etc.

In another embodiment to a hair treatment composition or skin care composition of the present invention, an amideamine compound and a neutralizing agent such as an organic and/or inorganic acid may be added together with a higher fatty acid and/or higher alcohol, so as to enhance the conditioning effect of the composition. The amount of an amideamine compound added is preferably 5% by weight or lower with respect to the weight (100% by weight) of the entire composition. If the amount in question added exceeded the above range, the skin treated with the resulting composition would become so mat and slimy as to displease the user.

Moreover, the conditioning effect may be enhanced by adding silicone (methylpolysiloxane, methylphenylsiloxane, high polymeric methylpolysiloxane, cyclic polysiloxane, polyether-modified silicone, amino-modified silicone, etd.) to a hair treatment composition or skin care composition of the present invention. The amount of a silicone added is preferably 5% by weight or lower with respect to the weight (100% by weight) of the entire composition. If the amount in question added exceeds the above range, the hairs or the skin treated with the resulting composition would become so mat and slimy as to displease the user, and moreover, the stability of the composition becomes deteriorated.

Other suitable components to be added to a hair treatment composition or to a skin care composition of the present invention may include cationic surfactants (alkyltrimethyl ammonium salts, dialkyldimethyl ammonium salts, alkylpyridium salts, alkyldimethylbenzyl ammonium salts, benzethonium chloride, benzalkonium chloride, etc.), solubilizers (ethanol, ethylene glycol, propylene glycol, etc.), waxes (carnauba wax, candelilla wax, etc.), hydrocarbon oils (liquid paraffin, squalane, etc.), moisture retainers (glycerin, trehalose, sorbitol, maltitol, dipropylene glycol, 1,3-butylene glycol, sodium hyaluronate, etc.), esters (hexyl laurate, isopropyl myristate, octyldodecyl myristate, myristic acid myristate, myristic acid-2-hexyldecyl, glycerin trimyristate, isopropyl palmitate, palmitic acid-2-heptylundecyl, palmitic acid-2-hexyldecyl, butyl stearate, isocetyl stearate, 12-hydroxystearic acid cholesteryl, cetostearylalcohol, cetyl octanate, hexyldecyldimethyl octanate, isocetyl isostearate, trimethylolpropane triisostearate, decyl oleate, oleic acid oil, cetyl lactate, myristyl lactate, ethyl acetate, butyl acetate/amyl acetate, acetic acid lanolin, 2-ethylhexanoic acid cetyl, 2-ethylhexyl palmitate, di-2-ethylhexylic acid ethylene glycol, tri-2-ethylhexylic acid trimethylol propane, tri-2-ethylhexylic acid glycerin, tetra-2-ethylhexylic acid pentaerythritol, cetyl-2-ethyl hexanoate, diisobutyl adipate, adipic acid-2-heptylundecyl, adipic acid-2-hexyldecyl, dipentaerythritol fatty acid ester, neopentyl glycol dicaprylate, diisostearyl malate, di-2-heptylundecanoic acid glycerin, tri-2-heptylundecanoic acid glyceride, caster oil fatty acid methyl ester, acetoglyceride, N-lauryl-L-glutamic acid-2-octyldodecyl ester, di-2-ethylhexyl sebacate, diisopropyl sebacate, succinic acid-2-ethylhexyl, triethyl citrate, ethyl laurate, mink oil fatty acid ester, etc.), antioxidants (tocopherol, BHT, etc.), higher alcohols, higher fatty acids (lauric acid, myristic acid, palmitic acid, stearic acid, behenic acid, isostearic acid, oleic acid, undecylenic acid, tall oil fatty acid, coconut oil fatty acid, palm oil fatty acid, palm kernel fatty acid, linolic acid, linoleic acid, eicosapentaenoic acid, docosahexanoic acid, etc.), amino acids (alginin, glutamic acid, etc.), UV absorbants (benzophenon derivatives, paraamino benzoate derivatives, methoxy cinnamate derivatives, etc.), UV-scattering agents (inorganic compounds such as zinc oxide, zirconium oxide, titanium oxide, etc.), thickeners, metal chelating agents (edetic acid salts, etc.), pH-adjusting agents, bactericides, preserving agents, hair growth stimulants, vitamins, antiinflammatory agents, dyes, pigments (inorganic white pigments such as titanium dioxide, inorganic red pigments such as iron oxide (Indian red), iron titanate, etc., inorganic green pigments such as cobalt titanate, etc., iron oxide-treated mica titanium, carbon black-treated mica titanium, etc.), perfumes, foaming-promoting agents, etc.

A cosmetic composition prepared according to the present invention as described above is not limited in prescription to any specific one, but may take any desired prescription. Furthermore, any appropriate additive usually used in a common cosmetic composition other than those derived above may be added to a cosmetic composition of the present invention, so long as it does not interfere with the effects originally expected from the composition, and the resulting composition may be processed by conventional techniques into appropriate products. Particularly, such an additive may be added with profit to a hair treatment composition of the present invention. Suitable prescriptions a hair treatment composition of the present invention can take include shampoos, hair rinses, hair conditioners, hair waxes, hair lotions, hair mists, etc. All these prescriptions are based on smoothness of a cation-modified purified galactomannan polysaccharide of the present invention added to the composition, the improvements of conditioning effects and finish feeling after use, and improvement of foaming quality. Since the cation-modified purified galactomannan polysaccharide of the present invention, when applied to the skin, can improve the texture of the skin, it is possible to use a skin care composition supplemented with the catrion-modified purified galactomannan polysaccharide of the present invention as a body cleanser, facial cleanser, or facial lotion. Furthermore, it may be added to acidic hair dye, oxidized hair dye or perm-setting agent. Many forumulation such as solid, dust, gel, cream, earzol, form may be used in accordance with portion or place to which the present invention is aplied.

EXAMPLE

The present invention will be detailed below by means of the preferred embodiments. However, the present invention is not limited to those embodiments. In the following description, the content of constituents in compositions expressed by "wt%" and "vol%" mean "percent by weight" and "percent by volume", respectively unless otherwise specified.

<u>Manufacture of cation-modified purified galactomannan polysaccharide</u>

Example 1

11.8 g of an aqueous 48 wt% sodium hydroxide solution was added to 900 ml of an aqueous 50 vol% isopropanol solution. 162 g of purified locust-bean gum containing 83 wt% of galactomannan was added and gradually dispersed to the mixture. 65.5 g of an aqueous 80 wt% glycidyltrimethyl ammonium chloride (GTA) solution was added to the mixture, and heated and reacted at 50˚C for three hours. After completion of the reaction, the reaction mixture was neutralized with 14.0 g of 50 vol% hydrochloric acid which was diluted with 1500 ml of an aqueous 50 vol% isopropanol solution. After neutralizing at room temperature for one hour, the reaction solution was poured into 1800 ml of methanol, and reaction product was precipitated and separated by filtration. The obtained precipitate was washed with an aqueous methanol solution, and the reaction product was dried under reduced pressure. Cationic charge density of the cation-modified purified galactomannan polysaccharide thus obtained was 0.95 meq/g. The result is shown in Table 1 (under

Sample 1 in Table 1).
Similarly, cation-modified purified galactomannan polysaccharides having different cationic charge density were synthesized by changing the amount of GTA to be added. The result is shown in Table 1 (under Samples 2 and 3 in Table 1).

Example 2

162 g of galactomannan-containing 88 wt% purified locust-bean gum was dispersed in 900 ml of an aqueous 55 vol% isopropanol solution and added with 48.3 g of an aqueous 48 wt% sodium hydroxide solution. Then, 142.8 g of 3-halogeno-2-hydroxypropyldimethylmonolauryl ammonium chloride was added to the mixture, heated and reacted at 50˚C for three hours. After completion of the reaction, the reaction mixture was neutralized with 14.0 g of 35% hydrochloric acid which was diluted with 1500 ml of an aqueous 70 vol% isopropanol solution. After neutralizing at room temperature for one hour, the reaction solution was poured into 1800 ml of methanol, and reaction product was precipitated and separated by filtration. The obtained precipitate was washed with an aqueous methanol solution, and the reaction product was dried under reduced pressure. Cationic charge density of the cation-modified purified galactomannan polysaccharide thus obtained was 0.75 meq/g. The result is shown in Table 1 (under Sample 4 in Table 1).

Example 3

In an autoclave, 162 g of the purified locust-bean gum obtained in Example 1 was dispersed in 900 mi of an aqueous 80 vol% isopropanol solution and added with 11.8 g of an aqueous 48 wt% sodium hydroxide solution. 66 g of ethylene oxide and 240 g of propylene oxide were added to the mixture and heated and reacted at 70˚C for three hours under pressurization and sealing. After completion of the reaction, pressurization was released and the reaction mixture was cooled to 50˚C. After cooling, 150 g of an aqueous 80 wt% GTA solution was added to the reaction mixture and reacted at 50˚C for three hours. After completion of the reaction, the reaction mixture was neutralized with 14.0 g of 35% hydrochloric acid which was diluted with 1500 ml of an aqueous 70 vol% isopropanol solution. After neutralizing at room temperature for one hour, the reaction solution was poured into 800 ml of methanol, and reaction product was precipitated and separated by filtration. The obtained precipitate was washed with an aqueous methanol solution, and the reaction product was dried under reduced pressure. Cationic charge density of the cation-modified purified galactomannan polysaccharide thus obtained was 0.83 meq/g. The result is shown in Table 1 (under Sample 5 in Table 1).

Example 4

11.8 g of an aqueous 48 wt% sodium hydroxide solution was added to 900 ml of an aqueous 50 vol% isopropanol solution. 162 g of purified locust-bean gum containing 97 wt% of galactomannan was gradually dispersed to the mixture. 77.2 g of an aqueous 80 wt% GTA solution was added to the mixture, and heated and reacted at 50˚C for three hours. After completion of the reaction, the reaction mixture was neutralized with 14.0 g of 35% hydrochloric acid which was diluted with 1500 ml of an aqueous 50 vol% isopropanol solution. After neutralizing at room temperature for one hour, the reaction solution was poured into 1800 ml of methanol, and reaction product was precipitated and separated by filtration. The obtained precipitate was washed with an aqueous methanol solution, and the reaction product was dried under reduced pressure. Cationic charge density of the cation-modified purified galactomannan polysaccharide thus obtained was 1.06 meq/g. The result is shown in Table 1 (under Sample 6 in Table 1).

Example 5

8.8 g of an aqueous 48 wt% sodium hydroxide solution and 3.0 g of sodium chloride was added to 900 ml of an aqueous 70 vol% isopropanol solution. 162 g of purified tara gum containing 84 wt% of galactomannan was gradually dispersed to the mixture. 44.1 g of an aqueous 80 wt% GTA solution was added to the mixture, and heated and reacted at 50˚C for three hours. After completion of the reaction, the reaction mixture was neutralized with 14.0 g of 35% hydrochloric acid which was diluted with 1500 ml of an aqueous 60 vol% isopropanol solution. After neutralizing at room temperature for one hour, the reaction solution was poured into 1800 ml of methanol, and reaction product was precipitated and separated by filtration. The obtained precipitate was washed with an aqueous methanol solution, and the reaction product was dried under reduced pressure. Cationic charge density of the cation-modified purified galactomannan polysaccharide thus obtained was 0.80 meq/g. The result is shown in Table 1 (under Sample 7 in Table 1).
Similarly, cation-modified purified galactomannan polysaccharides having different cationic charge density were synthesized by changing the amount of GTA to be added. The result is shown in Table 1 (under Samples 8 and 9 in Table 1).

Supplement 1

The same experimental procedures were repeated except that the added amount of GTA was varied, to produce some additional cation-modified purified galactomannan polysaccharides having different cationic charge densities. These results are also shown in Table 1 (under Samples 10 and 11 in Table 1).

Comparative Example 1

For the comparison with the cation-modified purified galactomannan polysaccharide of the present invention, cation-modified nonpurified galactomannan polysaccharide was synthesized by using galactomannan polysaccharide containing less than 80 wt% of galactomannan polysaccharides in crude galactomannan polysaccharides. Nonpurified locust-bean gum containing 74 wt% of galactomannan in crude galactomannan polysaccharides was cation-modified according to the method of Example 1. The cationic charge density of the obtained cation-modified nonpurified galactomannan polysaccharide was 0.91 meq/g. On the other hand, nonpurified tara gum containing 76 wt% of galactomannan in crude galactomannan polysaccharides was cation-modified according to the method of Example 5. The cationic charge density of the obtained cation-modified nonpurified galactomannan polyshaccaride was 0.88 meq/g. The result is shown in Table 1 (Samples 12 and 13 in Table 1).

TABLE 1. Cation Charge Density

| Sample No. | Charge density (meq/g) | Galactomannan content | Synthesis method | Sorts of polysaccharide |
|---|---|---|---|---|
| 1 | 0.95 | 83 wt% | Example 1 | purified locust bean gum |
| 2 | 0.42 | 83 wt% | Example 1 | purified locust bean gum |
| 3 | 2.48 | 83 wt% | Example 1 | purified locust bean gum |
| 4 | 0.75 | 88 wt% | Example 2 | purified locust bean gum |
| 5 | 0.83 | 83 wt% | Example 3 | purified locust bean gum |
| 6 | 1.06 | 97 wt% | Example 4 | purified locust bean gum |
| 7 | 0.80 | 84 wt% | Example 5 | purified tara gum |
| 8 | 1.25 | 84 wt% | Example 5 | purified tara gum |
| 9 | 2.61 | 84 wt% | Example 5 | purified tara gum |
| 10 | 0.05 | 83 wt% | Supplement 1 | purified locust bean gum |
| 11 | 4.10 | 83 wt% | Supplement 1 | purified locust bean gum |
| 12 | 0.91 | 74 wt% | Comparative Example 1 | nonpurified purified locast bean gum |
| 13 | 0.88 | 76 wt% | Comparative Example 1 | nonpurified tara gum |

[Production of Cosmetic Preparations Supplemented with Cation-modified Purified Galactomannan Polysaccharides, and their Evaluation] Example 6. Enhancement of the smoothness to hairs (Part 1)

Enhancement of the smoothness to hairs due to a cation-modified purified galactomannan polysaccharide was evaluated by means of a water-rinse hair cleansing preparation supplemented with the cation-modified purified galactomannan polysaccharide.

(Preparation of water-rinse hair cleansing preparation)

6-a

The cation-modified purified galactomannan polysaccharides prepared in Examples 1, 2 and 5 were used to prepare shampoo samples whose composition is as shown in column (A) of Table 2. The shampoo sample was prepared as follows. Distilled water (ingredient (12)) was heated to 80˚C to which one of the test Samples 1, 2 and 5 (ingredient (1)) as described above was slowly added with stirring and the mixture was stirred until it was dissolved. After recognizing the dissolution, heating was discontinued, and polyoxyethylene(3)lauryl ether sulfuric acid sodium (ingredient (5)), coconut oil fatty acid amide propylbetaine (ingredient (6)), and coconut oil fatty acid monoethanol amide (ingredient (7)) were added to the mixture with stirring at the weight ratios specified in Table 2. The mixture was stirred until it became a uniform liquid, to which sodium editate (ingredient (8)), sodium benzoate (ingredient (10)) and an aqueous solution of citric acid (for pH adjustment, pH 5.5-6.0)(ingredient (11)) were added at the weight ratios specified in Table 2 at 30 to 40˚C, and the mixture was stirred to homogeneity. Thus, three kinds of shampoo samples with compositions as shown under column (A) of Table 2 were obtained. The shampoo sample prepared from the cation-modified purified galactomannan polysaccharides represented by test sample 1 was named test prescription S1. The shampoo sample prepared from the cation-modified purified galactomannan polysaccharides represented by test Sample 4 was named test prescription S2. The shampoo sample prepared from the cation-modified purified galactomannan polysaccharides represented by test Sample 7 was named test prescription S3.

6-b

The cation-modified purified galactomannan polysaccharide obtained in Example 1 and named test Sample 1 and a cation-modified hydroxyethyl cellulose containing nitrogen at 1.8 wt% to which an average of 1.8 mol of ethylene oxide was introduced (Catinal HC-100, Toho Chemical Industry Co. Ltd.) and a copolymer of N-methacryloyloxyethyl N,N-dimethyl ammonium-α-methylcarboxylbetaine and alkyl methacrylate (Yukaformer SM, Mitsubishi Chemicals Co.) as an amphoteric water-soluble polymer were combined at the weight ratio specified in (B) in Table 2, and used as a test sample. To this test sample, ingredients shown under column (B) of Table 2 were added and the resulting product was used as a test shampoo sample. Specifically, distilled water (ingredient (12)) was heated to 80˚C to which one of the test Samples 1, 4 and 7 (ingredient (1)), the cationic water soluble polymer (ingredient (3)) and the amphoteric water-soluble polymer (ingredient (4)) were slowly added with stirring at the weight ratios specified in column (B) of Table 2. The stirring was continued until the substances added were dissolved. After recognizing the dissolution, heating was discontinued, and the ingredients (5) to (7) were added to the mixture with stirring at the weight ratios specified in Table 2. The mixture was stirred until it became a uniform liquid, to which ingredients (8), (10) and (11) were added at the weight ratios specified in Table 2 at 30 to 40˚C, and the mixture was stirred to homogeneity. A shampoo sample with a composition shown under column (B) of Table 2 was obtained. This was named a test prescription S4 of the present invention.

6-c (Preparation of comparative prescriptions)

To compare the effects of test shampoo preparations containing a cation-modified purified galactomannan polysaccharide of the present invention, comparative shampoo samples were prepared by using the cation-modified nonpurified galactomannan polysaccharides prepared in Comparative Example 1. The composition of the comparative shampoo samples was as shown under column (C) of Table 2. Specifically, distilled water (ingredient (12)) of the comparative shampoo sample in Table 2 was heated to 80˚C to which comparative Sample 12 or 13 (ingredient (2)) was slowly added with stirring. The stirring was continued until the substance added was dissolved. After recognizing the dissolution, heating was discontinued, and the ingredients (5) to (7) were added to the mixture with stirring. The mixture was stirred until it became a uniform liquid. Ingredients (8), (10) and (11) were added to the mixture at 30 to 40˚C, and the mixture was stirred to homogeneity. The shampoo sample compounded with cation-modified nonpurified galactomannan polysaccharide of the test Sample 12 were named comparative prescription C1. The shampoo sample compounded with cation-modified nonpurified galactomannan polysaccharide of the test Sample 13 were named comparative prescription C2.

TABLE 2. Shampoo Prescriptions

| Ingredients \ Weight ratio (%, in terms of solids) | | (A) | (B) | Comparative sample (C) |
|---|---|---|---|---|
| (1) | Present invention (test Samples 1, 4, 7) | 0.3 | | 0 |
| (2) | Comparative samples (comparative Sample 12, 13) | 0 | | 0.3 |
| (3) | Cationic water-soluble polymer (Catinal HC-100) | 0 | 0.3 | 0 |
| (4) | Amphoteric water-soluble polymer (Yukaformer SM) | 0 | 0.2 | 0 |
| (5) | Polyoxyethylene (3)lauryl ether sulfuric acid sodium | 9.0 | | 9.0 |
| (6) | Coconut oil fatty acid amide propylbetaine | 4.5 | | 4.5 |
| (7) | Coconut oil fatty acid monoethanol amide | 2.5 | | 2.5 |
| (8) | Sodium edetate | 0.1 | | 0.1 |
| (10) | Sodium benzoate | 0.1 | | 0.1 |
| (11) | Aqueous solution of citric acid (for pH adjustment, pH 5.5-6.0) | as appropriate | | as appropriate |
| (12) | Distilled water | rest | | rest |

(Evaluation)

Strands of hairs (manufactured by BEAULAX CO., LTD.; artificial hairs the roots of which were arranged uniformly; 60 mm in length and 40 mm in width) were immersed in 5% aqueous solutions of shampoo of the test prescriptions S1 to S4 prepared in the above sections 6-a to 6-b (present invention). After the shampoo was fully wahsed away, coefficient of dynamic friction was measured with respect to the hairs which were in wet-state as they were, by using a friction-sensing tester (KES-SE manufactured by KATO TECH CO., LTD.) to evaluate smoothness. The evaluation result is shown in Table 3. Strands of haris were similarly immpersed in shampoo of the comparative prescriptions C1 and C2 prepared in the above section 6-c. After the shampoo was fully wahsed away, coefficient of dynamic friction of the strands of hairs was measured with respect to the hairs which were in wet-state of hairs as they were, by the friction-sensing tester to evaluate smoothness. The result is shown in Table 3.

With respect to the shampoo of test prescriptions S1 to S4 prepared in the above sections 6-a to 6-c and the shampoo of the comparative prescriptions C1 and C2, ten testers carried out finger-passing text after hair-washing. After they washed and rinsed their hairs by using the shampoo of test prescriptions S1 to S4 and the shampoo of the comparative prescriptions C1 and C2 so as to determine finger-passing at rinsing time. Each of the prescriptions tested was ranked depending on the numbers of testers who gave good finger-passing according to the following four rank scale. The result is shown in Table 3.

◎ - The number of positive testers who felt good finger-passing is eight or more.
○ - The number of positive testers who felt good finger-passing is six to seven.
Δ - The number of positive testers who felt good finger-passing is four to five.
× - The number of positive testers who felt good finger-passing is three or less.

TABLE 3.

| Comparison of smoothness in water-rinse hair cleansing preparation (shampoo) | | | | | | |
|---|---|---|---|---|---|---|
| Cationic polymers used | Test prescriptions of the present invention | | | | Comparative prescriptions | |
| | S1 | S2 | S3 | S4 | C1 | C2 |
| | Sample 1 | Sample 4 | Sample 7 | Sample 1 + Catinal HC-100 + Yukaformer SM | Sample 12 | Sample 13 |
| Coefficent of dynamic property | 0.39 | 0.38 | 0.35 | 0.35 | 0.46 | 0.47 |
| Finger-passing | ◎ | ◎ | ◎ | ◎ | O | Δ |

From the result shown in Table 3, it was confirmed that the shampoos containing the cation-modified purified galactomannan polysaccharide of the present invention descended coefficient of dynamic friction in wet hairs in comparison with the shampoos containing the conventional cation-modified nonpurified galactomannan polysaccharides. This appears in organoleptic evaluation and it was confirmed that the shampoos of the cation-modified purified galactomannan polysaccharide of the present invention indicated excellent finger-passing after hair-washing in comparison with the shampoos containing the conventional cation-modified nonpurified galactomannan polysaccharides. Moreover, it is recognized that the above-mentioned effects were not lost even in the shampoos used in combination of cation-modified water-soluble polymer or amphoteric water-soluble polymer. It was confirmed that smoothness was improved by cation-modifying purified galactomannan polysccharide containing 80 wt% or more of galactomannan. Furthermore, since the improvement of smoothness to hairs was recognized, improvement of feeling to skin cosmetic compositions such as body cleansing agent, facial cleanser, etc.

Example 7. Smoothness to hairs (Part 2)

The smoothness affording effect on the damaged hairs by the cation-modified galactomannan purified polysaccharide of the present invention was tested in water-rinse hair cleansing preparations using shampoos containing the test prescriptions S1 to S4 of the present invention and comparative prescriptions C1 to C2 prepared in Example 6. The same strands of hairs as the one used in Example 6 were immersed in a bleaching solution which comprised a 6% $H_2O_2$ and 3% aqueous ammonium at 2:1 (w/w): the hair strand was immersed for 60 minutes in the bleaching solution whose weight against that of the hair strand was adjusted to 100:1 and whose temperature was kept at 40°C. The hair strand was rinsed with warm water, and dried with a dryer. The strands of hairs which had been seriously damaged by being immersed in the bleaching solution were immersed in aqueous 5% solution of shampoo containing the test prescriptions S1 to S4 of the present invention prepared in the above sections 6-a to 6-b. After shampoo was fully washed away, coefficient of dynamic friction was measured with respect to the hairs which were in wet-state as they are by using a friction-sensing tester to evaluate smoothness. The evaluation result is shown in Table 4. Strands of haris were similarly immpersed in shampoo of the comparative prescriptions C1 and C2 prepared in the above section 6-c. After the shampoo was fully wahsed away, coefficient of dynamic friction was measured with respect to the hairs which were in wet-state of hairs as they were by the friction-sensing tester to evaluate smoothness. The result is shown in Table 4.

Table 4

| Smoothness affording effect to damaged hairs in water-rinse hair cleansing preparation | | | | | | |
|---|---|---|---|---|---|---|
| Cationic polymers used | Test prescriptions of the present invention | | | | Comparative prescriptions | |
| | S1 | S2 | S3 | S4 | C1 | C2 |
| | Sample 1 | Sample 4 | Sample 7 | Sample 1 + Catinal HC-100+Yukaformer SM | Sample 12 | Sample 13 |
| Coefficent of dynamic property | 0.42 | 0.41 | 0.38 | 0.39 | 0.58 | 0.57 |

From the result shown in Table 4, it was confirmed that even in the damaged hairs, the prescriptions containing

cation-modified purified galactomannan polysaccharide obtained by cation-modifying purified galactomannan polysacchaide containing 80 wt% or more of galactomannan were excellent in effect of improving smoothness to damaged hairs in wet in comparison with prescriptions containing nonpurified cation-modified galactomannan polysaccharide. Moreover, the difference of coefficient of dynamic friction was become greater than the case evaluated in normal hairs which were not treated by bleaching solution in Example 6, and accordingly, it was confirmed that the present invention was useful to damaged hairs.

Example 8. Foaming test

Foaming activities were tested by using shampoos containing the test prescriptions S1 to S4 of the present invention prepared in the above sections 6-a to 6b of Example 6. For the comarison, foaming activities were similarly tested by using the comparative prescriptions C1 to C2 prepared in the above section 6-c of Example 6.

Testing procedures were as follows. A 175-ml sample of a 1.5% aqueous solution of each preparation shampoo of the prescriptions S1 to S4 of the present invention and of the comparative prescriptions C1 and C2. They were transmerred to a commercially available juice-mixer where it was spun to develop bubbles. The foam amount was plotted as a function of spun time. The results are shown in Table 5.

Table 5 Foaming test result (mL)

| Cationic polymers used | Test prescriptions of the present invention | | | | Comparative prescriptions | |
|---|---|---|---|---|---|---|
| | S1 | S2 | S3 | S4 | C1 | C2 |
| | Sample 1 | Sample 4 | Sample 7 | Sample 1 + Catinal HC-100 + Yukaformer SM | Sample 12 | Sample 13 |
| 1.5% aqueous solution | 1180 | 1170 | 1190 | 1190 | 1140 | 1150 |

From the result shown in Table 5, it was proved that the cation-modified purified galactomannan polysaccharides of the present invention were excellent in foaming activity in comparison with the conventional nonpurified cation-modified galactomannan polysaccharide. Accordingly, when the present invention is contained in a cosmetic composition having foam formation (lathering) such as a shampoo, body cleansers, facial cleansers, etc., foaming improvement can be expected.

In the following, improvement of smoothness, good foam-forming performance and feeling improvement which were obtained by the cation-modified purified galactomannan polysaccharide applied to normal hairs and damaged hairs were confirmed in various cosmetic compositions having different formulations.

<u>Hair shampoo</u>

(Preparation)

Example 9

9-a

The cation-modified galactomannan purified polysaccharides prepared in Examples 1 to 5 and named Samples 1-9 were used to prepare shampoo samples with compositions as shown in column (A) of Table 6. The shampoo sample was prepared as follows. Distilled water (ingredient (14)) in column (A) of Table 6 was heated to 65˚C to which one of the test Samples (ingredient (1)) was slowly added with stirring, and mixture was stirred until it was dissolved. After recognizing the dissolution, heating was discontinued, and polyoxyethylene(1,5)lauryl ether sulfuric acid triethanol amine (ingredient (5)), polyoxyethylene(3)lauryl ether sulfuric acid sodium (ingredient (6)), coconut oil fatty acid amide propylbetaine (ingredient (7)), coconut oil fatty acid monoethanol amide (ingredient (8)), dipropylene glycol (ingredient (9)) and ethylene glycol distearate (ingredient (10)) were added to the mixture with stirring. The mixture was stirred until it became a uniform liquid, to which sodium edetate (ingredient (11)), sodium benzoate (ingredient (12)), and an aqueous solution of citric acid (for pH adjustment, pH 5.5-6.0)(ingredient (13)) were added, and the mixture was stirred to homogeneity. Thus, five kinds of shampoo samples with compositions as shown under column (A) of Table 6 were obtained. The five shampoo samples containing the cation-modified purified galactomannan polysaccharides represented by

Samples 1-9 were named test prescriptions 1-9 of the present invention, respectively.

9-b

The cation-modified purified galactomannan polysaccharide prepared in Example 1 and named Sample 1 and a cation-modified hydroxyethyl cellulose containing nitrogen at 1.8 wt% to which an average of 1.8 mol of ethylene oxide was introduced (Catinal HC-100, Toho Chemical Industry Co., Ltd.) represented as a cation-modified water-soluble polymer A in Table 6 were combined at the weight ratios specified in Table 6, and used as a test sample. To this test sample, ingredients shown under column (B) of Table 6 were added and the resulting product was used as a test shampoo sample. Specifically, distilled water (ingredient (14)) was heated to 65˚C to which Sample 1 (ingredient (1)) and cationic water-soluble polymer A or B (ingredient (3)) were slowly added with stirring at the weight ratios specified in column (B) of Table 6. The stirring was continued until the substances added were dissolved. After recognizing the dissolution, heating was discontinued, and the ingredients (5) to (10) were added to the mixture with stirring. The mixture was stirred until it became a uniform liquid, to which ingredients (11) to (13) were further added at 30 to 40˚C, and the mixture was stirred to homogeneity. A shampoo sample with a composition as shown under column (B) of Table 6 was obtained. This was named test prescription 10.

9-c

The same procedures as in the above section 9-b were employed except that a copolymer of diallyldimethyl ammonium chloride and acryl amide (Merquat 550, Nalco) was used instead of the cation-modified water-soluble polymer A, to provide a shampoo sample with a composition as shown under column (C) of Table 6. This was named test prescription 11.

9-d

The cation-modified purified galactomannan polysaccharide prepared in Example 1 and named Sample 1 and a copolymer of N-methacryloyloxyethyl N,N-dimethyl ammonium-α-methylcarboxylbetaine and alkyl methacrylate (Yukaformer SM, Mitsubishi Chemicals Co.) as an amphoteric water-soluble polymer were combined at the weight ratio specified in Table 6, and used as a test sample. To this test sample, ingredients shown under column (D) of Table 6 were added and the resulting product was used as a test shampoo sample. Specifically, distilled water (ingredient (14)) was heated to 65˚C to which one of Samples 1 to 5 (ingredient (1)) and the amphoteric water-soluble polymer (ingredient (4)) were slowly added with stirring at the weight ratios specified in column (D) of Table 6. The stirring was continued until the substances added were completely dissolved. After recognizing the dissolution, heating was discontinued, and the ingredients (5) to (10) were added to the mixture with stirring. The mixture was stirred until it became a uniform liquid. Ingredients (11) to (13) were added to the mixture kept at 30 to 40˚C, and the mixture was stirred to homogeneity. A shampoo sample with a composition as shown under column (D) of Table 6 was obtained. This was named test prescription 12.

9-e

The cation-modified galactomannan polysaccharide prepared in Example 1 and named Sample 1, the cationic water-soluble polymer and the amphoteric water-soluble polymer were combined at the weight ratios specified under (E) in Table 6, and used as a test sample. To this test sample, ingredients shown under column (E) of Table 6 were added and the resulting product was used as a test shampoo sample. Specifically, distilled water (ingredient (14)) under (E) of Table 6 was heated to 65˚C to which one of Samples 1 to 5 (ingredient (1)), the cationic water-soluble polymer A or B (ingredient (3)), and the amphoteric water-soluble polymer (ingredient (4)) were slowly added with stirring at the weight ratios specified in column (E) of Table 6. The stirring was continued until the substances added were dissolved. After recognizing the dissolution, heating was discontinued, and the ingredients (5) to (10) were added to the mixture with stirring. The mixture was stirred until it became a uniform liquid. Ingredients (11) to (13) were added to the mixture at 30-40˚C, and the mixture was stirred to homogeneity. A shampoo sample with a composition as shown under column (E) of Table 6 was obtained. This was named test prescription 13.

9-f (Preparation of comparative prescriptions)

To compare the effects of the test shampoo preparations containing the cation-modified purified galactomannan polysaccharide of the present invention, comparative shampoo samples were prepared by using the cation-modified purified galactomannan polysaccharides prepared in Supplement 1 and listed in Table 1 under the names Samples 10

and 11. The composition of the comparative shampoo samples was as shown under column (G) of Table 6. Specifically, distilled water (ingredient (14)) was heated to 65°C to which Samples 10, 11, 12 or 13 (ingredient (2) in column (G) of Table 6) was slowly added with stirring. The stirring was continued until the substance added was dissolved. After recognizing the dissolution, heating was discontinued, and the ingredients (5) to (10) were added to the mixture with stirring. The mixture was stirred until it became a uniform liquid. Ingredients (11) to (13) were added to the mixture at 30 to 40°C, and the mixture was stirred to homogeneity. The shampoo samples thus obtained were named comparative prescriptions 1 and 2.

9-g (Preparation of comparative prescriptions)

To further compare effects, shampoos having the composition shown under comparative prescription (G) in Table 6 were prepared in similairty with the above section 9-f by using, instead of cation-modified purified galactomannan polysaccharide in the above section 9-f, cation-modified nonpurified galactomannan polysaccharide in which cation-modifying nonpurified galactomannan polysaccharide containing less than 80 wt% of galactomannan was cation-modified, i.e., Samples 12 and 13 in Table 1 which were obtained in Comparative Example 1. The obtained products were named as comparative prescriptions 3 and 4.

TABLE 6. Prescription of Shampoo Preparations

| Ingredients \ Weight ratio (%, in terms of solids) | | (A) | (B) | (C) | (D) | (E) | Reference prescription (F) | Comparative prescriptions (G) |
|---|---|---|---|---|---|---|---|---|
| (1) | Present invention (samples 1-5) | 0.6 | | | | | 0 | 0 |
| (2) | Comparative samples (sample 10-13) | 0 | | | | | 0 | 0.6 |
| (3) | Cationic water-soluble polymer A (Catinal HC-100) | 0 | 0.4 | 0 | 0 | 0.2 | 0 | 0 |
| | Cationic water-soluble polymer B (Merquat 550) | 0 | 0 | 0.4 | 0 | 0 | 0 | 0 |
| (4) | Amphoteric water-soluble polymer (Yukaformer SM) | 0 | 0 | 0 | 0.4 | 0.2 | 0 | 0 |
| (5) | Polyoxylethylene(1,5)laurylether sulfuric acid triethanol amine | 6.0 | | | | | 6.0 | 6.0 |
| (6) | Polyoxyethylene(3)laurylether sulfuric acid sodium | 3.0 | | | | | 3.0 | 3.0 |
| (7) | Coconut oil fatty acid amide propylbetaine | 4.5 | | | | | 4.5 | 4.5 |
| (8) | Coconut oil fatty acid monoethanol amide | 2.5 | | | | | 2.5 | 2.5 |
| (9) | Dipropylene glycol | 3.0 | | | | | 3.0 | 3.0 |
| (10) | Ethylene glycol distearate | 1.0 | | | | | 1.0 | 1.0 |
| (11) | Sodium edetate | 0.1 | | | | | 0.1 | 0.1 |
| (12) | Sodium benzoate | 0.1 | | | | | 0.1 | 0.1 |
| (13) | Aqueous solution of citric acid (for pH adjustment, pH 5.5-6.0) | as appropriate | | | | | as appropriate | as appropriate |
| (14) | Distilled water | rest | | | | | rest | rest |

9-h (Preparation of comparative prescription)

To further compare the effects with other cationic, a comparative shampoo sample was prepared by using, instead of the cation-modified nonpurified galactomannan polysaccharide in the above section 9-f, a cation-modified guar gum, in which the composition ratio of mannose and galactose is 2 : 1 though it is the same galactomannan polysaccharide, containing nitrogen at 1.9 wt% (Catinal CG-100, Toho Chemical Industry Co., Ltd.). The ingredients shown under column (G) of Table 6 were treated in the same manner as described in the above section 9-f to provide the comparative shampoo preparation which was named comparative prescription 5.

(Evaluation)

Example 10

In this test, test prescriptions 1 to 13 prepared as described in the above sections 9-a to 9-e, and a reference prescription were used. The reference prescription with a composition as shown under column (F) of Table 6 was obtained by using ingredients similar to those used for the production of test and comparative prescriptions and treating them similarly, except that they did not contain any cationic polymer.

Of test prescriptions 1 to 13, and the reference prescription, ten testers washed their hair using each of the prescriptions, and dried the hair with a dryer. The testers compared the conditioning effects of the respective test prescriptions as described below during and after use with those of the reference prescription. The conditioning effects compared consisted of:

* foam-formation (lathering) at washing
* smoothness during use (finger-passing and feeling of hair)
* comb-passing feeling after use (dried hairs).

The tester's evaluation was scored according to the following five rank scale. For each of the test prescriptions, the scores of the ten testers were summed as in shown in Table 7, and the sum of ten testers' evaluation was taken as its overall evaluation result. The evaluation results of the test prescriptions are shown in Table 8. The reference prescription (F) was prepared in such a manner that distilled water (ingredient (14)) was heated to 65˚C to which the ingredients (5) to (10) were added. The mixture was stirred until it became a uniform liquid. To the mixture which was cooled to 30 to 40˚C, ingredients (11) to (13) were added, and the mixture was stirred to homogeneity.

Comparative Example 2

With regard to comparative prescriptions 1 to 5 prepared as described in 9-f to 9-h, their conditioning effects were compared with those of the reference prescription in the same manner as in Example 10. The thus obtained evaluation results of comparative prescriptions 1 to 5 are shown in Table 8.

TABLE 7. Ranking Scale for Evaluating Test Prescriptions

| | Test properties | | | |
|---|---|---|---|---|
| | During use | | | After use |
| Score | Foam formation (Lathering) | Easy finger-passing at washing | Feeling of wet hair | Easy comb-passing |
| +2 | Higher | Better | Smoother | Better |
| +1 | Slightly higher | Sighlty better | Slightly smoother | Slightly better |
| 0 | Same | Same | Same | Same |
| -1 | Slightly lower | Slightly worse | Slightly more sticky | Slightly rigidity |
| -2 | Lower | Worse | More sticky | Rigidity |

TABLE 8. Evaluation Results of Shampoo Preparations

| | | Cationic polymer used | | During use | | | After use |
|---|---|---|---|---|---|---|---|
| | | | | Foam-formation (Lathering) | Easy finger passing at wasing | Feeling of wet hairs | Easy comb-passing |
| Test prescip-tion | 1 | Sample 1 | | 16 | 17 | 16 | 15 |
| | 2 | Sample 2 | | 16 | 15 | 15 | 15 |
| | 3 | Sample 3 | | 16 | 16 | 15 | 14 |
| | 4 | Sample 4 | | 15 | 17 | 17 | 16 |
| | 5 | Sample 5 | | 16 | 16 | 16 | 14 |
| | 6 | Sample 6 | | 17 | 17 | 17 | 15 |
| | 7 | Sample 7 | | 17 | 16 | 16 | 16 |
| | 8 | Sample 8 | | 17 | 17 | 16 | 15 |
| | 9 | Sample 9 | | 15 | 16 | 16 | 15 |
| | 10 | Sample 1 | CatinalHC-100 | 17 | 17 | 17 | 16 |
| | 11 | | Merquat 550 | 17 | 16 | 16 | 17 |
| | 12 | | Yukaformer SM | 16 | 16 | 16 | 16 |
| | 13 | | Catinal HC-100 and Yukaformer SM | 17 | 17 | 16 | 16 |
| Comparative prescription | 1 | Sample 10 | | 5 | -10 | -8 | 1 |
| | 2 | Sample 11 | | -2 | -7 | -6 | -6 |
| | 3 | Sample 12 | | 9 | 10 | 10 | 10 |
| | 4 | Sample 13 | | 10 | 11 | 9 | 9 |
| | 5 | Catinal CG-100 | | 8 | 11 | 9 | 5 |

From the results of Table 8, it was confirmed that when the cation-modified purified galactomannan polysaccharides of the present invention prepared by cation-modifying purified galactomannan polysaccharide were added to a shampoo preparation, foam-formation (lathering) at washing hairs was improved and conditioning effects such as finger-passing and feeling during use were superior, and conditioning effects of comb-passing after hair-dried were superior.

On the other hand, the comparative prescriptions 3 and 4 containing samples 12 and 13 prepared by cation-modifying nonpurified galactomannan polysaccharide were worse in conditioning effects such as "finger-passing at washing", etc. in comparison with the cation-modified purified galactomannan polysaccharides of the present invention, and were poor in smoothness and finish-feeling after use. From these facts, it was shown that the performance was improved by the cation-modified purified galactomannan polysaccharides of the present invention prepared by cation-modifying purified galactomannan polysaccharide containing 80 wt% or more of galacttomannan. Moreover, by using, together, a cationic water-soluble polymer (Catinal HC-100, Merquat 550) and/or an amphoretic water-soluble polymer (Yukaformer SM) as the compounding ingredients, the conditioning effects of the test prescriptions were found to be enhanced without disturbing the performance possesed by the cation-modified purified galactomannan polysaccharide of the present invention. It was confirmed that when the test prescriptions are compared with the comparative prescription 5 which contains a cationic polymer (cation-modified guar gum: Catinal CG-100) prepared by cation-modifying galactomannan polysaccharide in which composite ratio of mannose and galactose is 2 : 1, the present invention surpassed in both "during use" and "after use" and confered smoothness at wasing hairs and finish-feeling after hair dry which the cation-modified purified galactomannan polysaccharide of the present invention has, in comparison with the conventional conditioning agents which have not such smoothness and finish-feeling.

(Exemplary shampoo compositions 1-5)

Preferred shampoo compositions according to the present invention will be described below.

TABLE 9. Composition 1. Shampoo (1)

| Ingredient | Weight ratio (%) |
|---|---|
| Plyoxyethylene(3)lauryl ether sulfonic acid sodium | 6.0 |
| Coconut oil fatty acid amide methyl taurine sodium | 3.0 |
| Sodium Lauroamphoacetate | 3.0 |
| Lauric acid propylene glycol | 0.9 |
| Coconut oil fatty acid diethanol amide | 1.0 |
| Polyoxyethylene(4)alkyl(C12-14)sulfosuccinic acid disodium | 2.0 |
| Cationic cellulose | 0.5 |
| Present invention (Sample 2) | 0.2 |
| Propylene glycol | 0.3 |
| Sodium chloride | 0.5 |
| Sodium benzoate | 0.3 |
| Disodium 2 edetate | 0.05 |
| Purified water | rest |
| Perfume | 0.3 |

Purified water was heated to 70°C, to which other ingredients were added and dissolved to homogeneity. The mixture was then cooled.

TABLE 10. Composition 2. Shampoo (2)

| Ingredient | Weight ratio (%) |
|---|---|
| Distearic acid ethylene glycol | 2.0 |
| Coconut oil fatty acid amide methyl taurine sodium | 8.0 |

(continued)

| Ingredient | Weight ratio (%) |
|---|---|
| Perm kernel fatty acid amide propylbetaine | 5.0 |
| Present invention (Sample 1) | 0.5 |
| Laurylhydroxysulfobetaine | 1.5 |
| Coconut oil fatty acid amide monoethanolamide | 3.0 |
| Stearic acid dimethylaminopropyl amide | 0.3 |
| Citric acid | 0.55 |
| Sodium chloride | 1.2 |
| Phenoxyethanol | 0.1 |
| Perfume, pigment, preservative | as appropriate |
| Metal ion sealant, pH adjuster | as appropriate |
| Purified water | rest |

Purified water was heated to 70˚C, to which other ingredients were added and dissolved to homogeneity. The mixture was then cooled.

TABLE 11. Composition 3. Shampoo (3)

| Ingredients | Weight ratio ((%) |
|---|---|
| Polyoxyethylene(2)laurylether sulfuric acid sodium | 10.0 |
| Polyoxyethylene coconut oil fatty acid monoethanolamide sulfuric acid sodium | 2.0 |
| Distearic acid ethylene glycol | 1.8 |
| Lauric acid propylene glycol | 2.0 |
| Coconut oil fatty acid amide propylbetaine | 4.0 |
| Methylpolysiloxane micro-emulsion | 1.0 |
| Present invention (Sample 6) | 0.3 |
| Polyquaternium-7 | 0.1 |
| Propylene glycol | 2.0 |
| Coconut oil fatty acid amide diethanolamide | 0.5 |
| Glyceryl oleate | 0.2 |
| Citric acid | 0.8 |
| Alginine | 0.1 |
| Sodium chloride | 0.4 |
| Phenoxyethanol | 0.1 |
| Sodium benzoate | 0.3 |
| Disodium edetate | 0.05 |
| Purified water | rest |
| Perfume | as appropriate |

Purified water was heated to 70˚C, to which other ingredients were added and dissolved to homogeneity. The mixture was then cooled.

TABLE 12. Composition 4. Shampoo (4)

| Ingredient | Weight ratio (%) |
|---|---|
| Present invention (Sample 1) | 0.3 |
| Polyquaternium-7 | 0.1 |
| Cation-modified fenugreek gum (*1) | 0.2 |
| Distearic acid ethylene glycol | 2.0 |
| Coconut oil fatty acid amide methyl taurine sodium | 5.0 |
| Coconut oil fatty acid amide sarcosine sodium | 3.0 |
| Perm kernel fatty acid amide propylbetaine | 5.0 |
| Laurylhydroxysulfobetaine | 1.5 |
| Coconut oil fatty amide monoethanolamide | 3.0 |
| Octenylsuccinic acid dextrin ester | 0.5 |
| Stearic acid dimethylaminopropyl amide | 0.3 |
| Citric acid | 0.55 |
| Sodium chloride | 1.2 |
| Phenoxyethanol | 0.1 |
| Perfume, pigment, preservative | as appropriate |
| Metal ion sealant, pH adjuster | as appropriate |
| Purified water | rest |

Purified water was heated to 70˚C, to which other ingredients were added and dissolved to homogeneity. The mixture was then cooled.
*1 Cation-modified fenugreen gum (INCI name: Trigonella Foenum-Graecum Hydroxypropyltrimonium Chloride)

(INCl name: International Nomenclature Cosmetic Ingredient name)

TABLE 13. Composition 5. Shampoo (5)

| Ingredient | Weight ratio (%) |
|---|---|
| Present invention (Sample 2) | 0.1 |
| Present invention (Sample 8) | 0.3 |
| Cation-modified guar gum *2 | 0.2 |
| Distearic acid ethylene glycol | 2.0 |
| Coconut oil fatty acid amide methyl taurine sodium | 6.0 |
| Polyoxyethylene coconut oil fatty acid monoethanolamide sulfuric acid sodium | 3.0 |
| Coconut oil fatty acid amide propylbetaine | 5.0 |
| Lauryl dimethylamino acetic acid betaine | 1.5 |
| Coconut oil fatty acid diethanolamide | 3.0 |
| Highly polymerized methyl polysiloxane | 0.2 |
| Polyoxyethylene(4)laurylether | 0.2 |
| Sodium chloride | 1.2 |

(continued)

| Ingredient | Weight ratio (%) |
|---|---|
| Phenoxyethanol | 0.1 |
| Perfume, pigment, preservative | as appropriate |
| Metal ion sealant, pH adjuster | as appropriate |
| Purified water | rest |

Purified water was heated to 70˚C, to which other ingredients were added and dissolved to homogeneity. The mixture was then cooled. *2 Cation-modified guar gum (INCI name: Guar Hydroxypropyltrimonium Chloride)

As a result that effects in the costmetic compositions prepared according to composition examples 1 to 5 were confirmed, foam-formation (lathering) and foam quality during washing hairs were improved, conditioning effects such as finger-passing during hair-washing and during rinsing and feeling were excellent, and further, conditioning effects such as comb-passing and feeling after hair-drying.

Hair Rinse

(Preparation)

Example 11

11-a

The cation-modified purified galactomannan polysaccharides prepared in Examples 1, 2, 3 and 5 and named Samples 1, 4, 5 and 7 were used to prepare hair rinse samples whose composition is as shown in column (A) of Table 14 below. The hair rinse sample contained an amideamine compound, a behenic acid methylaminopropylamide citric acid salt which had been neutralized by citric acid as a neutralizer, and a higher alcohol (cetanol). The hair rinse sample was prepared as follows. Ingredients (5) to (9) and (11) specified in column (A) of Table 14 were heated to 80˚C and stirred until the mixture became a uniform liquid. One of the test Samples 1, 4, 5 and 7 (ingredient (1)) was previously added with stirring to distilled water (ingredient (13)), and the mixture was stirred until it was dissolved. The solution, after being heated to 80˚C, was poured into the above liquid with stirring, and an aqueous solution of citric acid (ingredient (12)) was added to the mixture while the mixture was being cooled, and the mixture was stirred until it became a uniform solution. Thus, hair rinse sample with compositions as shown under column (A) of Table 14 were obtained. The hair rinse samples containing the cation-modified purified galactomannan polysaccharides of the present invention represented by samples 1, 4, 5 and 7 were named test prescriptions 14 to 17.

11-b

The cation-modified purified galactomannan polysaccharides prepared in Example 1 and named Sample 1 and a cation-modified hydroxyethyl cellulose containing nitrogen at 1.8 wt% into which an average of 1.8 mol of ethylene oxide was introduced (Catinal HC-100, Toho Chemical Industry Co., Ltd.) were combined at the weight ratios specified in Table 14 to give a mixture to which ingredients shown under column (B) of Table 14 were added, and the resulting product was used as a test hair rinse sample. Specifically, ingredients (5) to (9) and (11) specified in column (B) of Table 14 were heated to 80˚C and stirred until the mixture became a uniform liquid. Ingredient (1) and Catinal HC-100 (ingredient (3)) were previously added with stirring to distilled water (ingredient (13)), and the mixture was stirred until it was dissolved. The solution, after being heated to 80˚C, was poured into the above liquid with stirring, and the mixture was stirred until it became a uniform solution. Then, an aqueous solution of citric acid (ingredient (12)) was added to the mixture while the mixture was being cooled. The obtained sample was named test prescription 18.

11-c

The cation-modified purified galactomannan polysaccharides prepared in Example 1 and named Sample 1 and a copolymer of N-methacryloyloxyethyl N,N-dimethyl ammonium-$\alpha$-methylcarboxylbetaine and alkyl methacrylate (Yukaformer SM, Mitsubishi Chemicals Co.) as an amphoteric water-soluble polymer were combined into a mixture to which ingredients shown under column (C) of Table 14 were added and the resulting product was used as a test hair rinse sample. Ingredients (5) to (9) and (11) specified in column (C) of Table 14 were heated to 80˚C and stirred until

the mixture became a uniform liquid. Ingredient (1) and Yukaformer SM (ingredient (4)) were previously added with stirring to distilled water (ingredient (13)), and the mixture was stirred until it was dissolved. The solution, after being heated to 80˚C, was poured into the above liquid with stirring, and the mixture was stirred until it became a uniform solution. Then, an aqueous solution of citric acid (ingredient (12)) was added to the mixture while the mixture was being cooled. The mixture was stirred until it became a uniform solution. The obtained sample was named test prescription 19.

11-d

The cation-modified purified galactomannan polysaccharides prepapred in Exampel 1 and named Sample 1 were used to prepare hair rinse samples whose composition is as shown in column (D) of Table 14 below. The hair rinse sample contained an amideamine compound, a behenic acid dimethylaminopropylamide citric acid salt which had been neutralized by citric acid as a neutralizer, a higher alcohol (cetanol) and a silicone (methylpolysiloxane). The hair rinse sample was prepared as follows. Ingredients (5) to (11) specified in column (D) of Table 14 were heated to 80˚C and stirred until the mixture became a uniform liquid. Ingredient (1) was previously added with stiring to distilled water (ingredient (13)), and the mixture was stirred until it was dissolved. The solution, after being heated to 80˚C, was poured into the above liquid with stirring, and the mixture was stirred until it became a uniform solution. Then, an aqueous solution of citric acid (ingredient (12)) was added to the mixture while the mixture was being cooled. The obtained sample was named test prescription 20.

11-e (Preparation of comparative prescriptions)

To compare the effects of the test hair rinse preparations containing the cation-modified purified galactomannan polysaccharide of the present invention, comparative shampoo samples were prepared by using the cation-modified purified galactomannan polysaccharides prepared in Supplement 1 and listed in Table 1 under the names samples 10 and 11. The composition of the comparative hair rinse samples was as shown under column (F) of Table 14. Specifically, ingredients (5) to (9) and (11) specified in column (F) of Table 14 were heated to 80˚C and stirred until the mixture became a uniform liquid. Ingredient (2) was previously added with stirring to distilled water (ingredient (13)), and the mixture was stirred until it was completely dissolved. The solution, after being heated to 80˚C, was poured into the above liquid with stirring, and the mixture was stirred until it became a uniform solution. Then, an aqueous solution of citric acid (ingredient (12)) was added to the mixture while the mixture was being cooled. The mixture was stirred until it became a uniform solution. Thus, hair rinse samples containing Samples 10 and 11 in Table 1 were obtained. These samples were named comparative prescriptions 6 and 7, respectively.

11-f (Preparation of comparative prescription)

To further compare effects, rinses having the composition shown under comparative prescription (F) in Table 14 were prepared in similarity with the above section11-e by using, instead of the Samples 10 and 11, cation-modified nonpurified galactomannan polysaccharide in which nonpurified galactomannan polysaccharide was cation-modified, i.e., Samples 12 and 13 in Table 1 which were obtained in Comparative Example 1. The obtained rinses were named as comparative prescriptions 8 and 9.

TABLE 14. Prescription of Rinse Preparations

| Ingredient \ Weight ratio (%) in terms of solids) | | (A) | (B) | (C) | (D) | Reference prescription (E) | Comparative prescription (F) |
|---|---|---|---|---|---|---|---|
| (1) | Present invention (Samples 1, 4, 5 and 7) | 1.0 | | | | 0 | 0 |
| (2) | Comparative samples (Samples 10, 11, 12, 13) | 0 | | | | 0 | 1.0 |
| (3) | Cationic water-soluble polymer (Catinal HC-100) | 0 | 0.5 | 0 | 0 | 0 | 0 |
| (4) | Amphoeric water-soluble polymer (Yukafomer SM) | 0 | 0 | 0.5 | 0 | 0 | 0 |
| (5) | Behenic acid dimethyamino-propylamide citric acid salt | 2.0 | | | | 2.0 | 2.0 |
| (6) | Cetyl lactate | 2.0 | | | | 2.0 | 2.0 |
| (7) | Polyoxyethylene(4)-stearylether | 1.0 | | | | 1.0 | 1.0 |
| (8) | Isopropyl palmitate | 1.0 | | | | 1.0 | 1.0 |
| (9) | Cetanol | 6.0 | | | | 6.0 | 6.0 |
| (10) | Methylpolysiloxane | 0 | 0 | 0 | 0.8 | 0 | 0 |
| (11) | Paraoxybenzoic acid methyl | 0.1 | | | | 0.1 | 0.1 |
| (12) | Aqueous solution of citric acid (for pH adjustment, pH 4.0-4.5) | as appropriate | | | | as appropriate | as appropriate |
| (13) | Distilled water | rest | | | | rest | rest |

(Evaluation)

Example 12

In this test, performance evaluation shown in the following items was conducted with respect to the test prescriptions 14 to 20 prepared as described in the above sections 11-a to 11-d (rinse) by using ten testers. Performance evaluation system used rinse having composition shown under a reference prescription (E) in Table 14 containing no cationic polymer in its ingredient and rinses to be evaluated.

Of test prescriptions 10 to 15, and the reference prescription having no cationic polymer, ten testers washed their hair using each of the prescriptions, and dried the hair with a dryer. The testers compared use feeling of finger-passing during rinsing and of drying by use of a dryer with respect to the respective test prescriptions with those of the reference prescription (E).

* smoothness during rinsing (finger-passing)

*existence of conditioning effects of dried hairs (comb-passing and frictional feel)

The tester's evaluation was scored according to the following five rank scale in Table 15. For each of the test prescriptions, the scores of the ten testers were summed. The evaluation results of the test prescriptions are shown in Table 15. Ingredients (5) to (11) specified in column (E) of Table 14 were heated to 80˚C and stirred until the mixture became a uniform liquid. Distilled water (ingredient (13)), after being heated to 80˚C, was poured into the above liquid with stirring, and the mixture was stirred until it became a uniform solution. Then, an aqueous solution of citric acid (ingredient (12)) was added to the mixture while the mixture was being cooled. The mixture was stirred until it became a uniform solution.

Comparative Example 3

With regard to comparative prescriptions 6 to 9 prepared as described in 11-e and 11-f, their conditioning effects were compared with those of the reference prescription in the same manner as in Example 12. The thus obtained evaluation results of comparative prescriptions 6 to 9 are shown in Table 16.

TABLE 15. Ranking Scale for Evaluating Test Prescriptions

| | Test properties | | |
|---|---|---|---|
| Score | Finger-passing | Comb-passing | Frictional feel |
| +2 | Better | Better | Smaller |
| +1 | Slightly better | Slightly better | Slightly smaller |
| 0 | Same | Same | Same |
| -1 | Slightly worse | Slightly worse | Slightly larger |
| -2 | Worse | Worse | Slightly larger |

TABLE 16. Evaluation Results of Rinse Preparations

| | | Test prescriptions | | | | | | | Comparative Prescriptions | | | |
|---|---|---|---|---|---|---|---|---|---|---|---|---|
| Test cationic polymers | | 14 | 15 | 16 | 17 | 18 | 19 | 20 | 6 | 7 | 8 | 9 |
| | | Sample 1 | Sample 4 | Sample 5 | Sample 7 | Sample 1 | | Sample 1 | Sample 10 | Sample 11 | Sample 12 | Sample 13 |
| | | | | | | Catinal HC-100 | Yukaformer SM | | | | | |
| Finger-passing at rinsing | | 16 | 17 | 16 | 16 | 17 | 17 | 16 | 0 | -2 | 10 | 9 |
| At dried | Comb-passing | 16 | 16 | 16 | 17 | 15 | 15 | 16 | -4 | -3 | 9 | 10 |
| | Frictional feel | 13 | 14 | 13 | 13 | 14 | 13 | 15 | 0 | 2 | 9 | 9 |

From the results of Table 16, it was confirmed that the hair rinse preparations which contained, in addition to the cation-modified purified galactomannan polysaccharide of the present invention which was prepared by cation-modifying purified galactomannan polysaccharide containing 80 wt% or more of galactomannan, an amideamine compound, neutralizer and higher alcohol (cetanol) were excellent in smoothness of the compositions at rinsing. Moreover, since comb-pasing after drying hairs was improved and conditioning effects such as less frictional feel enabled to be obtained, it was confirmed good finish feel by the present invention in the systems containing an amideamine compound, neutralizer and higher alcohol.

Furthermore, when the performance of the test hair rinse preparations (Test prescriptions 14 to 20) containing the amideamine compound, neutralizer and higher alcohol were compared with nonpurified cation-modified galactomannan polysaccharides prepared by cation-modifying nonpurified galactomannan polysaccharide containing less than 80 wt% of galactomannan (Comparative prescriptions 8 to 9) of the Samples 12 and 13, it was confirmed that the present compositions were excellent in both conditioning effects during use and finish feel after use. This means that the catinon-modified purified galactomannan polysaccharide of the present invention reduces precipitates derived from outer cover, proteins and water-unsoluble substances in comparison with the conventional nonpurified cation-modified galactomannan polysaccharide prepared by cation-modifying nonpurified galactomannan polysaccharide containing less than 80 wt% of galactomannan, whereby good smoothness during rinse treatment was obtained and at the same time, finish feel after use enabled to be improved. Moreover, it was confirmed that by using, together, cationic water-soluble polymer and/or amphoeric water-soluble polymer, the conditioning effects of the test prescriptions were found to be enhanced without disturbing the performance possessed by the cation-modified purified galactomannan polysaccharide of the present invention. Furthermore, it was confirmed that by containing silicone such as methylpolysiloxane, etc., conditining effects enabled to be improved without disturbing the performance possessed by the cation-modified purified galactomannan polysaccharide of the present invention.

(Exemplary hair rinse compositions 6-9)

Preferred hair treatment compositions such as hair rinses, hair conditioners, etc., according to the invention which require conditioning effects will be described below.

TABLE 17. Composition 6. Rinse (1)

| Ingredients | Weight ratio (%) |
|---|---|
| Olive oil | 0.3 |
| Stearyl alcohol | 2.5 |
| Cetyl alcohol | 2.7 |
| Glycerin | 4.0 |
| 1,3-butylene glycol | 5.0 |
| Hydrolyzed wheat | 0.2 |
| Hydroxystearic acid | 0.5 |
| 2-ethylhexanoic acid cetyl | 1.0 |
| Distearyldimethylammonium chloride | 0.2 |
| Hydroxypropyltrimonium chloride starch | 0.3 |
| Behenic acid dimethylaminopropylamide | 0.5 |
| Present invention (Sample 4) | 1.0 |
| L-glutamic acid | 0.5 |
| Silicone oil | 2.0 |
| Perfume, Pigment, Preservative | as appropriate |
| Purified water | rest |

To purified water in Table 17, the cation-modified purified galactomannan polysaccharide of the present invention, hydroxypropyltrimonium chloride starch, behenic acid dimethylaminopropylamide, hydrolyzed wheat, L-glutamic acid, glycerin, 1,3-butylene glycol, and the mixture was maintained at 80˚C (water phase). The remaining ingredients in Table 17 were combined and heated so that they were dissolved, and the mixture was maintained at 80˚C (oil phase). The water phase was combined with the oil phase, and the mixture was spun with a homogenizer to be emulsified. Then, the emulsion was cooled with stirring.

TABLE 18. Composition 7. Rinse (2)

| Ingredients | Weight ratio (%) |
|---|---|
| Isopropyl palmitate | 3.0 |
| Cetyl lactate | 3.0 |
| Behenyl alcohol | 2.0 |
| Stearyl alcohol | 2.0 |
| Hydroxypropyltrimonium hydrolyzed keratin | 0.3 |
| Stearyltrimethylammonium chloride | 0.7 |
| Glycerin | 0.45 |
| Isocetyl myristate | 0.1 |
| Mink fat fatty acid ethyl | 0.2 |
| Self-emulsified monostearic acid glycerin | 0.2 |
| Present invention (Sample 3) | 1.2 |

(continued)

| Ingredients | Weight ratio (%) |
|---|---|
| Copolymer of aminoethylaminopropylsiloxane and dimethylsiloxane | 2.0 |
| Dimethylpolysiloxane | 0.5 |
| Phenoxyethanol | 0.5 |
| Pigment | as appropriate |
| Perfume | as appropriate |
| Purified water | rest |

To purified water in Table 18, the cation-modified purified galactomannan poiysaccharide of the present invention, stearyltrimethylammonium chloride, glycerin, hydroxypropyltrimonium hydrolyzed keratin, phenoxyethanol and pigment were added, and the mixture was maintained at 75˚C (water phase). The remaining ingredients in Table 18 were combined and heated so that they were dissolved, and the mixture was maintained at 75˚C (oil phase). The water phase was combined with the oil phase, to which isopropanol was added, and the mixture was spun with a homogenizer to be emulsified. Then, the emulsion was cooled with stirring.

TABLE 19. Composition 8. Rinse (3)

| Ingredients | Weight ratio (%) |
|---|---|
| Present invention (Sample 3) | 0.3 |
| Present invention (Sample 7) | 0.4 |
| Polyquaternium-7 | 0.2 |
| Polyquaternium-22 | 0.1 |
| Octyl palmitate | 3.0 |
| Cetyl lactate | 3.0 |
| Behenyl alcohol | 2.0 |
| Cetanol | 1.5 |
| Stearyltrimethylammonium chloride | 1.0 |
| Glycerin | 0.45 |
| Isocetyl myristate | 0.1 |
| Mink fat fatty acid ethyl | 0.2 |
| Polyoxyethylene(4)stearylether | 0.5 |
| Dimethylpolysiloxane | 2.0 |
| Glyceryl stearate | 1.0 |
| Phenoxyethanol | 0.5 |
| Pigment, Perfume | as appropriate |
| Purified water | rest |

Rinse was prepared by a conventional method.

TABLE 20. Composition 9. Conditioner

| Ingredients | Weight ratio (%) |
|---|---|
| Cetanol | 1.5 |
| Behenyl alcohol | 4.0 |

(continued)

| Ingredients | Weight ratio (%) |
|---|---|
| 1,3-butylene glycol | 1.0 |
| Glycerin | 2.0 |
| 2-ethylhexanoic acid cetyl | 2.0 |
| Isocetyl myristate | 0.4 |
| Mink fat fatty acid ethyl | 0.1 |
| Polyoxyethylene(4)stearylether | 1.0 |
| N-(3-alkyl(12,14)oxy-2-hydroxypropyl)-L-alginic hydrochloride | 0.6 |
| Present invention (Sample 8) | 0.6 |
| Stearic acid dimethylaminopropylamide | 1.5 |
| 50% lactic acid | 0.9 |
| Phenoxyethanol | 0.3 |
| Sodium benzoate | 0.3 |
| Perfume | 0.5 |
| Purified water | rest |

To purified water in Table 20 cation-modified purified galactomannan polysaccharide of the present invention, N-(3-alkyl(12, 14)oxy-2-hydroxypropyl)-L-alginic hydrochloride, Stearic acid dimethylaminopropylamide, 50% lactic acid, glycerin, phenoxyethanol and sodium benzoate were added, and the mixture was maintained at 70˚C (water phase). The remaining ingredients were combined and heated so that they were dissolved, and the mixture was maintained at 70˚C (oil phase). The water phase was combined with the oil phase, and the mixture was spun with a homogenizer to be emulsified. Then, the emulsion was cooled with stirring.

As a result that effects in the costmetic compositions prepared according to composition examples 6 to 9 were confirmed, smoothness during rinse treatment was excellent, comb-passing after hair-dry was improved glating feeling was less and accordingly, conditioning effects were exellent.

Hair Color

(Preparation)

Example 13

13-a

The cation-modified purified galactomannan polysaccharide prepared in Example 1 and 5 and named Samples 1 and 7 were used to prepare two-component oxidation hair color samples whose composition was as shown in column (A) of Table 21 below. The two-component oxidation hair color samples containing the cation-modified purified galactomannan polysaccharides of the Samples 1 and 7 were named test prescriptions 21 and 22, respectively. At the time of use, the first and second components of each hair color sample was mixed at 1:1 weight ratio, and the mixture was applied to the hair.

13-b (Preparation of comparative prescriptions)

To compare the effects of the cation-modified purified galactomannan polysaccharide of the present invention in hair color preparations, a comparative hair color sample was prepared by using the nonpurified cation-modified galactomannan polysaccharide obtained in Comparative Example 1 in which nonpurified galactomannan polysaccharide containing less than 80 wt% of galactomannan content, i.e., Sample 13 in Table 1, to which ingredients specified under column (C) of Table 21 were added to provide a two-component oxidation hair color sample. The two-component oxidation hair color sample which contained nonpurified cation-modified tara gum of Sample 13 in Table 1 was named

comparative prescription 10. At the time of use, the first and second components of the hair color sample was mixed at 1:1 weight ratio, and the mixture was applied to the hair.

TABLE 21. Prescription of Combinational Oxidation Hair Color

| Ingredients \ Weight ratio (%, in terms of solids) | | | (A) | Reference prescription (B) | Comparative prescription (C) |
|---|---|---|---|---|---|
| 1st component | (1) | Test prescriptions (Samples 1, 7) | 0.5 | 0 | 0 |
| | (2) | Comparative preparation (Sample 13) | 0 | 0 | 0.5 |
| | (3) | Cetostearylalcohol | 5.0 | | |
| | (4) | POE(5)behenylether | 2.0 | | |
| | (5) | POE(20)stearylether | 2.0 | | |
| | (6) | Highly polymerized silicone | 0.2 | | |
| | (7) | Propylene glycol | 2.0 | | |
| | (8) | Liquid paraffin | 5.0 | | |
| | (9) | 28% aqueous ammonium solution | 2.0 | | |
| | (10) | Toluene-2,5-diamine | 1.5 | | |
| | (11) | Resorcin | 1.0 | | |
| | (12) | Disodium edetate | 0.2 | | |
| | (13) | Purified water | rest | | |
| 2nd component | (1) | Aqueous hydrogenperoxide solution (35%) | 17.0 | | |
| | (2) | Cetanol | 2.0 | | |
| | (3) | POE(30)cetylether | 3.0 | | |
| | (4) | POE(2)laurylether sulfuric acid sodium | 0.3 | | |
| | (5) | Methylparaben | 0.1 | | |
| | (6) | Citric acid (pH 3.5) | as appropriate | | |
| | (7) | Disodium hydrogenphosphate | 0.1 | | |
| | (8) | Disodium edetate | 0.2 | | |
| | (9) | Purified water | The rest | | |

(Evaluation)

Example 14

In this test, performance evluation shown in the following items was conduced with respect to the test prescriptions 21 and 22 prepared as described in the above section13-a, and a reference prescription were used. The reference prescription had a composition as shown under column (B) of Table 21. Preparation of the reference prescription occurred as in the test prescriptions, except that it did not contain any cationic polymer.

For each of the test prescriptions, the first and second components were combined in equal amounts prior to use, and the mixture was applied to the hair. The hair was left at room temperature for 30 minutes, rinsed for 3 minutes with running water kept at 40˚C, and dried with a drier.

Ten testers participated to evaluate the performance of the test prescriptions as compared with the reference prescription (B) with special attention being paid to:

* smoothness of the hairs during rinsing, and
* feeling after hair-dry.

The tester's evaluation was scored according to the following five rank scale in Table 22.

For each of the test prescriptions, the scores of the ten testers were summed and the sum was taken as its

overall evaluation result (in Table 22). The evaluation results of the test prescriptions are shown in Table 23. In that time, first and second components of each of hair color samples were mixed at 1:1 wieght ratio, and the mixture was applied to hair.

Comparative Example 4

With regard to comparative prescription 10 prepared as described in the above section 13-b, its performance was compared with that of the reference prescription in the same manner as in Example 14. The thus obtained evaluation results are shown in Table 23.

TABLE 22. Ranking Scale for Evaluating Test Prescriptions

| | Test properties | |
|---|---|---|
| Score | Smoothness during rinsing | Finish feel after drying hairs |
| +2 | Better | Better |
| +1 | Slightly better | Slightly better |
| 0 | Same | Same |
| -1 | Slightly worse | Slightly worse |
| -2 | Worse | Worse |

TABLE 23. Evaluation Results of Combinational Hair Color Prescriptions

| Test cationic polymers | Test prescriptions | | Comparative prescriptions |
|---|---|---|---|
| | 21 | 22 | 10 |
| | Sample 1 | Sample 7 | Sample 13 |
| Smoothness during rinsing | 14 | 13 | 5 |
| Finish feel after drying hairs | 12 | 11 | 4 |

The results of Table 23 indicate that the cation-modified purified galactomannan polysaccharide of the present invention, when added to a hair color preparation, enhances the smoothness of the hairs during rinsing which contributed to the treatment of damaged hairs, and enabled easy finger-passing. It also improved finish feel after drying hairs. Thus, it was confirmed the cation-modified purified galactomannan polysaccharide of the invention brought about beneficial effects when added to a hair color composition.

Further, it was confirmed that when the test prescriptions are compared with comparative prescription 10, the former preparations 21 and 22 of the present invention ensured more improved smoothness during rinsing treatment finish feel after drying of hairs in comparison with the comparative prescription 10 which contained the nonpurified cation-modified galactomannan polysaccharide, and they treated more effectively damaged hairs in comparison with the conventional nonpurified cation-modified galactomannan polysacchardies.

(Exemplary two-component hair color compositions 10 and 11)

The following shows preferred hair color compositions which utilize the conditioning effects by the cation-modified purified galactomannan polysaccharide of the present invention on damaged hairs.

TABLE 24. Composition 10. Prescription of Combinational Oxidation Hair Color

| Ingredients | | | Weight ratio (%) |
|---|---|---|---|
| 1st component | (1) | Test prescriptions (Samples 2) | 0.3 |
| | (2) | Polyquaternium-6 | 0.15 |
| | (3) | Polyquaternium-10 | 0.15 |
| | (4) | Stearyltrimethylammonium chloride | 1.0 |
| | (5) | POE(20)oleylether | 5.0 |
| | (6) | POE(10)hexyldecilether | 10.0 |
| | (7) | Liquid amidopropylbetaine laurate | 5.0 |
| | (8) | Cetanol | 5.0 |
| | (9) | Propylene glycol | 15.0 |
| | (10) | Polyethylene glycol | 5.0 |
| | (11) | Sodium thioglycolate | 0.5 |
| | (12) | Aminopropyldimethicone | 0.4 |
| | (13) | Toluene 2,5-diamine | 3.0 |
| | (14) | Resorcin | 0.8 |
| | (15) | 2,4-diaminophenoxyethanol hydrogen chloride | 0.2 |
| | (16) | Paraphenylene diamine | 0.2 |
| | (17) | Metha-aminophenol | 0.2 |
| | (18) | 28% aqueous ammonium solution | 5.0 |
| | (19) | Sodium sulfite | 0.5 |
| | (20) | Perfume | as appropriate |
| | (21) | Preservative | as appropriate |
| | (22) | Purified water | rest |
| 2nd component | (1) | Aqueous hydrogenperoxide solution (35%) | 17.0 |
| | (2) | Cetanol | 1.0 |
| | (3) | Sodium laurylsulfate | 0.3 |
| | (4) | Phenacetin | 0.1 |
| | (5) | Disodium edetate | 0.2 |
| | (6) | Purified water | rest |

Prescription of combinational oxidation hair color was prepared by a conventional method.

TABLE 25. Compositon 11. Prescription of Combinational Hair-Decoloring

| Ingredients | | | Weight ratio (%) |
|---|---|---|---|
| 1st component | (1) | Test prescription (Samples 7) | 1.5 |
| | (2) | Aqueous solution of hydrolyzed collagen (36.5%) | 3.0 |
| | (3) | Coconut oil fatty acid amidopropyl betaine | 30.0 |
| | (4) | POE(2)dodecilether | 15.0 |
| | (5) | POE(10)cetylether | 15.0 |
| | (6) | Aminopropyldimethicone | 0.3 |
| | (7) | 2-octyldecanol | 4.5 |
| | (8) | Polyethylene glycol | 5.0 |
| | (9) | Trimethylbehenylammonium chloride | 1.0 |
| | (10) | 28% aqueous ammonium solution | 2.0 |
| | (11) | Ascorbic acid | 0.5 |
| | (12) | Anhydrous sodium sulfite | 0.2 |
| | (13) | Perfume | as appropriate |
| | (14) | Preservative | as appropriate |
| | (15) | Purified water | rest |
| 2nd component | (1) | Aqueous hydrogenperoxide solution (35%) | 15.0 |
| | (2) | Cetanol | 2.0 |
| | (3) | POE(20)cetylether | 3.0 |
| | (4) | Sodium larylsulfate | 0.5 |
| | (5) | Preservative | 0.1 |
| | (6) | glycolic acid | as appropriate |
| | (7) | Disodium hydrogenphosphate | 0.1 |
| | (8) | Disodium edetate | 0.2 |
| | (9) | Purified water | rest |

Prescription of combinational hair-decoloring color was prepared by a conventional method.

As a result that effects in the costmetic compositions prepared according to composition examples 10 to 11 were confirmed, smoothness during rinse treatment was excellent and finish feel after drying hairs were exellent, and conditioning effects were excellent.

(Compositions 12 to 13)

Preferable composition examples of the present invention which are applied to other hair-treatment compositions which require conditioning effects are shown in the following.

TABLE 26. Composition 12. Hair Wax

| Ingredients | Weight ratio (%) |
|---|---|
| Present invention (Sample 1) | 0.3 |
| Cation-modified guar gum (*3) | 0.1 |
| Polyquaternium-6 | 0.1 |
| Polyquaternium-28 | 0.3 |
| Liquid paraffin | 10.0 |

(continued)

| Ingredients | Weight ratio (%) |
|---|---|
| Micro-crystalline wax | 10.0 |
| Methylpolysiloxane | 5.0 |
| Stearyl alcohol | 3.0 |
| Propylene glycol | 10.0 |
| Carnauba wax | 3.0 |
| Isostearic acid | 1.0 |
| Stearic acid | 5.0 |
| Tetra 2-ethylenehexanic acid pentaelisulit | 2.0 |
| Polyoxyethylene hardened castor oil | 3.0 |
| Polyoxyethylenelaurylether phosphoric acid | 2.0 |
| Self-emulsified monostearic acid glyceryl | 2.0 |
| Triethanolamine | 1.0 |
| D-δ-tocophenol | 0.05 |
| Oat extract | 0.1 |
| Paraoxybenzoic acid ester | 0.2 |
| Polyacrylic acid sodium | 0.05 |
| Purified water | rest |
| Perfume | 0.08 |

Hair wax was prepared by a conventional method.
*3 Cation-modified guar gum (INCl name: Guar Hydroxypropyltrimonium Chloride)

TABLE 27. Composition 13. Hair Gel

| Ingredients | Weight ratio (%) |
|---|---|
| Present invention (Sample 3) | 0.8 |
| Polyquaternium-7 | 0.2 |
| Polyquaternium-11 | 1.0 |
| Glycerin | 5.0 |
| Ethanol | 20.0 |
| Polyoxyethyleneoctyldodecylether | 1.0 |
| Perfume, Chelating agent | as appropriate |
| Purified water | rest |

Hair gel was prepared by a conventional method.

Body Cleansing Preparation

(Preparation)

Example 15

15-a

The cation-modified cation-modified purified galactomannan polysacchardie prepared in Examples 1, 2 and 5, and named Samples 1, 4 and 7 were used to prepare body cleansing samples (body soaps) whose composition was as shown in column (A) of Table 28 below. Specifically, distilled water (ingredient (11)) was heated to 60˚C, to which one of the test Samples 1, 4 and 7 (ingredient (1)) was added with stirring, and the mixture was further stirred until it became a uniform solution. Then, ingredients (3) to (7) were added to the solution at 50 to 60˚C with stirring and the mixture was further stirred until it was completely dissolved. Similarly, ingredients (8) to (10) were added to the mixture at 30-40˚C with stirring and dissolved to homogeneity. Thus, three kinds of body cleansing samples with compositions as shown under column (A) of Table 28 were obtained. The three body cleansing samples containing the cation-modified purified galactomannan polysaccharide represented by Samples 1, 4 and 7 were named test prescriptions 23, 24 and 25, respectively.

TABLE 28. Prescription of Body Cleansers (Body Soaps)

| Ingredients \ Weight ratio (%, in terms of solids) | | (A) | Reference prescription (B) | Comparative prescriptions (C) |
|---|---|---|---|---|
| (1) | Test prescriptions (Sample 1, 4, 7) | 0.4 | 0 | 0 |
| (2) | Comparative samples (Sample 12, Cational HC-100) | 0 | 0 | 0.4 |
| (3) | Lauryl sulfuric acid ammonium | 9.5 | 9.5 | 9.5 |
| (4) | Polyoxyethylene(3)laurylether sulfuric acid sodium | 2 | 2 | 2 |
| (5) | Polyoxyethylene(2)laurylether phosphoric acid sodium | 4 | 4 | 4 |
| (6) | Coconut oil fatty acid amide propylbetaine | 3 | 3 | 3 |
| (7) | Coconut oil monoethanol amide | 2.7 | 2.7 | 2.7 |
| (8) | Disodium edetate | 0.2 | 0.2 | 0.2 |
| (9) | Sodium benzoate | 0.2 | 0.2 | 0.2 |
| (10) | Aqueous solution of citric acid (for pH adjustment, pH 5.7-6.2) | appropriate | appropriate | appropriate |
| (11) | Distilled water | rest | rest | rest |

15-b (Preparation of comparative prescription)

To compare the effects of the cation-modified purified galactomannan polysaccharide of the present invention with those of a conventional cationic polymer in body cleansing preparations, the Sample 12 obtained in the Comparative Example 1 and another comparative body cleansing samples as other cationic polymer were prepared by using a cation-modified hydroxyethyl cellulose containing nitrogen at 1.8 wt% into which an average of 1.8 mol of ethylene oxide was introduced (Catinal HC-100, Toho Chemical Industry Co., Ltd.), and adding thereto ingredients specified under column (C) of Table 28. Specifically, distilled water (ingredient (11)) was heated to 60˚C, to which Catinal HC-100 (ingredient (2)) was slowly added with stirring and dissolved. After recognizing the dissolution, ingredients (3) to (7) were added to the solution at 50 to 60˚C with stirring and the mixture was further stirred until it was dissolved to homogeneity. Then, ingredients (8) to (10) were added to the solution at 30 to 40˚C with stirring in the same manner as above, until they were dissolved to homogeneity. Thus, body cleansing samples with a composition as shown under column (C) of Table

28 were obtained. The samples were named comparative prescriptions 11 and 12.

(Evaluation)

Example 16

In this test, test prescriptions 23, 24 and 25 prepared as described in the above section 15-a and a reference prescription were used. The reference prescription had a composition as shown under column (B) of Table 28, the reference prescription containing no high polyer such as cationic polymer, etc. in this ingredients.

Each of the test prescriptions was used by ten testers to wash their body. The testers evaluated the performance of the test prescriptions as compared with the reference prescription with special attention being paid to:

*foam amount and foam quality during use,
*use feeling during use (easy rinsing, no tautness and sliminess after rinsing), and
*use feeling after use (after dried) (no tautness, smoothness, and moist feeling).

The tester's evaluation was scored according to the following five rank scale.

For each of the test prescriptions, the scores of the ten testers were summed in Tables 29 and 30 and the sum was taken as its overall evaluation result by the system sthon in Table 31.

Distilled water (ingredient (11)) was heated to 60˚C, to which ingredients (3) to (7) were added at 50 to 60˚C with stirring and dissolved to homogeneity. To the resulting solution at 30 to 40˚C ingredients (8) to (10) were added with stirring and the mixture was further stirred until it was completely dissolved. Thus, a body cleansing sample with a composition as shown under column (B) of Table 28 was obtained. The sample was used as a reference in this test.

TABLE 29. Ranking Scale for Evaluating Test Prescriptions (During Use)

| | Test properties | |
|---|---|---|
| Score | Foam amount | Foam quality |
| +2 | Higher | Better |
| +1 | Slightly higher | Slightly better |
| 0 | Same | Same |
| -1 | Slightly lower | Slightly worse |
| -2 | lower | Worse |

| | Test properties | | |
|---|---|---|---|
| Score | Rinsing easiness | Tautness after rinsing | Sliminess after rinsing |
| +2 | Easier | Less taut | Less slimy |
| +1 | Slightly easier | Slightly less taut | Slightly less slimy |
| 0 | Same | Same | Same |
| -1 | Slightly harder | Slightly more taut | Slightly more |
| -2 | Harder | More taut | More slimy |

TABLE 30. Ranking Scale for Evaluating Test Prescriptions (After Use)

| | Test properties | | |
|---|---|---|---|
| Score | Tautness after dried | Smoothness after dried | Moist feeling after dried |
| +2 | Less taut | Smoother | More moisture-rich |
| +1 | Slightly less taut | Slightly smoother | Slightly more moisture-rich |
| 0 | Same | Same | Same |
| -1 | Slightly more taut | Slightly more sticky | Slightly more chapping |

(continued)

| | Test properties | | |
|---|---|---|---|
| Score | Tautness after dried | Smoothness after dried | Moist feeling after dried |
| -2 | More taut | More sticky | More chapping |

Comparative Example 5

With regard to comparative prescriptions 11 and 12 prepared as described in the above section15-b, their performance was compared with that of the reference prescription in the same manner as in Example 16. The thus obtained evaluation results are shown in Table 31.

TABLE 31. Evaluation Results of Body Wash Prescriptions

| | | Test prescriptions | | | Comparative prescriptions | |
|---|---|---|---|---|---|---|
| | | 23 | 24 | 25 | 11 | 12 |
| Test cationic polymers | | Sample 1 | Sample 2 | Sample 4 | Sample 12 | Cation-modified hydroxyethyl cellulose (Cational HC-100) |
| During use | Foam amount | 16 | 15 | 17 | 13 | 10 |
| | Foam quality | 15 | 16 | 15 | 12 | 8 |
| | Rinsing easiness | 12 | 13 | 12 | 8 | -2 |
| | Tautness after rinsing | 11 | 10 | 10 | 7 | 5 |
| | Sliminess after rinsing | 10 | 9 | 10 | 7 | -6 |
| After use | Tautness after dry | 11 | 12 | 11 | 5 | -1 |
| | Smoothness after dry | 9 | 9 | 11 | 6 | -3 |
| | Moist feeling after dry | 10 | 11 | 11 | 7 | 5 |

From the results of Table 31, it was confirmed that when the inventive cation-modified purified galactomannan polysaccharide was added to a body cleansing preparation, use feeling at using time was improved and the foam amount and foam quality were improved, and finis feeling after use was improved. Moreover, when the present invention was compared with the comparative prescription 12 conaining the conventional nonpurified cation-modified galactomannan polysaccharide, the present invention was excellent in use feeling, foam quality, foam amount and feeling after use. From these facts, it was shown that the performance of the body cleansing preparation was improved by the cation-modified purified galactomannan polysaccharide of the present invention obtained by cantion-modifying purified galactomanna polysaccharide containing 80 wt/% or more of galactomann. Moreover, when the present invention was compared with another cationic polymer (comparative prescription 12) with respect to performance during use and after use,

it was confirmed that the cation-modified purified galactomann polysaccharide of the present invention was excellent.

(Compositions 14 to 18)

Preferable composition examples of cosmetic compositions such as body cleansers in which conditioning effects and improvement of feeling by removing water-unsoluble substances in the cation-modified purified galactomannan polysaccharide of the present invention were utilized are shown below.

TABLE 32. Composition 14. Body Cleanser (1)

| Ingredients | Weight ratio (%) |
|---|---|
| Dipropylene glycol | 2.5 |
| Disodium sulfosuccinic laurylate | 2.0 |
| Polyoxypropylene glycerylether | 1.0 |
| Isostearic acid | 1.0 |
| Lauric acid | 2.0 |
| Palm fatty acid | 9.0 |
| Myristic acid | 10.0 |
| Ethylene glycol distearate | 1.0 |
| Coconut oil fatty acid diethanolamide | 1.0 |
| Polyoxyethylene(2)laurylether sulfuric acid sodium | 3.0 |
| Coconut oil fatty acid sarcosine sodium | 2.0 |
| Aqueous solution of potassium hydroxide (47%) | 10.0 |
| Present invention (Sample 7) | 0.4 |
| Disodium edetate | 0.1 |
| Sodium benzoate | 0.1 |
| Purified water | rest |
| Perfume | as appropriate |

Liquid body cleansing preparation (body soap) were prepared by a conventional method.

TABLE 33. Composition 15. Body Cleanser (2)

| Ingredients | Weight ratio (%) |
|---|---|
| Polyoxyethylene(2)laurylether phosphate | 12.0 |
| Polyoxyethylene(3)laurylether sulfuric acid sodium | 3.0 |
| Coconut oil fatty acid taurine sodium | 2.0 |
| Coconut oil fatty acid diethanolamide | 1.0 |
| Ethyleneglycol distearate | 1.5 |
| Coconut oil fatty acid amide propylbetaine | 3.0 |
| Propylene glycol | 3.5 |
| 1,3-butylene glycol | 0.5 |
| Triethanolamine | 4.3 |
| Present invention (Sample 1) | 0.2 |
| Polyquaternium-10 | 0.05 |

(continued)

| Ingredients | Weight ratio (%) |
|---|---|
| Glycerin | 0.1 |
| Disodium edetate | as appropriate |
| Preservative, Perfume | as appropriate |
| Purified water | rest |

Liquid body cleansing preparation (body soap) were prepared by a conventional method.

TABLE 34. Composition 16. Body Cleanser (3)

| Ingredients | Weight ratio (%) |
|---|---|
| POE alkyl(12-14) sulfosuccinic acid disodium | 6.0 |
| Coconut oil fatty acid methyl taurine sodium | 9.0 |
| Laurylhydroxy sulfobetaine | 2.3 |
| Coconut oil fatty acid amide propylbetaine | 1.7 |
| Coconut oil fatty acid diethanolamide | 3.1 |
| Ethyleneglycol distearate | 3.0 |
| Present invention (Sample 6) | 0.2 |
| Cetanol | 0.5 |
| Disodium edetate | 0.2 |
| Paraoxybenzoic acid ester | 0.2 |
| Citric acid, perfume | as appropriate |
| Purified water | rest |

Liquid body cleansing preparation (body soap) were prepared by a conventional method.

TABLE 35. Composition 17. Facial Cleanser (1)

| Ingredients | Weight ratio (%) |
|---|---|
| Present invention (Sample 4) | 0.14 |
| Present invention (Sample 8) | 0.06 |
| Polyquaternium-7 | 0.2 |
| Polyquaternium-10 | 0.55 |
| Glycerin | 4.0 |
| Squalane | 1.0 |
| 25% aqueous solution of coconut oil fatty acid taurine sodium | 17.0 |
| Myristic acid | 7.2 |
| Palmitic acid | 10.8 |
| Polyoxyethylene(8)laurylether | 2.0 |
| Ethyleneglycol distearate | 3.0 |
| Polyoxyethylene(2)laurylether phosphate | 9.0 |
| 30% aqueous solution of coconut oil fatty acid amide propylbetaine | 5.0 |
| Aqueous solution of potassium hydroxide (47%) | 15.3 |

(continued)

| Ingredients | Weight ratio (%) |
|---|---|
| Crystalline cellulose | 0.1 |
| Menthol | 0.2 |
| Disodium edetate | 0.2 |
| Paraoxybenzoic acid methyl | 0.2 |
| Purified water | rest |
| Perfume | as appropriate |

A facial cleansing preparation was prepared by a conventional method.

TABLE 36. Composition 18. Facial Cleanser (2)

| Ingredients | Weight ratio (%) |
|---|---|
| Present invention (Sample 1) | 0.4 |
| Polyquaternium-6 | 0.16 |
| Polyquaternium-39 | 0.2 |
| Glycerin | 2.2 |
| Sorbitol | 0.8 |
| 25% aqueous solution of coconut oil fatty acid glutamic acid potassium | 10.0 |
| 30% aqueous solution of coconut oil fatty acid taurine sodium | 20.0 |
| Ethyleneglycol distearate | 3.0 |
| 30% aqueous solution of coconut oil fatty acid amide propylbetaine | 15.0 |
| Glyceryl mono-oleate | 2.0 |
| Ceteares-60 myristyl glycol | 2.0 |
| 10% aqueous solution of citric acid | 20.0 |
| Alginine | 3.0 |
| Disodium edetate | 15.0 |
| Phenoxyethanol | 2.0 |
| Purified water | 2.0 |
| Perfume | as appropriate |

A facial cleansing preparation was prepared by a conventional method.

As a result that effects in the costmetic compositions prepared according to the composition examples 14 to 18 were confirmed, improved effect of use feeling and foam qualtiy were confimred and improvement of finish feel after use was confirmed.

(Exemplary skin treatment compositions 19-22)

As other preferred examples which utilize the feeling improvement by the cation-modified purified galactomannan polisaccharide of the present invention, compositions such as after-shave lotions, etc. will be described below.

TABLE 37. Composition 19. After-shave lotion

| Ingredients | Weight ratio (%) |
|---|---|
| Carboxyvinyl polymer | 0.3 |

(continued)

| Ingredients | Weight ratio (%) |
|---|---|
| Isodecil oleate | 8.0 |
| 2-hexyldecil palmitate | 3.0 |
| Polyoxyethylenecetylether(18) | 3.0 |
| Octyl dodecanol | 4.0 |
| Glycerin | 3.0 |
| Cetanol | 1.0 |
| Ethanol | 3.0 |
| Present invention (Sample 9) | 1.0 |
| Paraoxybenzoic acid methyl | 0.2 |
| Squalane | 0.2 |
| Hydrolyzed silk | 0.2 |
| Menthol | as appropriate |
| Pigment | as appropriate |
| Perfume | as appropriate |
| 25% sodium hydroxide (for pH adjustment, pH5.7-6.2) | as appropriate |
| Distilled water | rest |

Liquid after-shave lotion was prepared by a conventional method.

TABLE 38. Composition 20. Make-up lotion

| Ingredients | Weight ratio (%) |
|---|---|
| Present invention (Sample 6) | 0.05 |
| Present invention (Sample 7) | 0.05 |
| Nonpurified cation-modified tara gum (*4) | 0.1 |
| Polyquaternium-7 | 0.4 |
| Glycerin | 0.7 |
| Squalane | 0.3 |
| 1,3-butanediol | 2.0 |
| Ethyl alcohol | 6.0 |
| Polyoxyethylene(60) hardened castor oil | 0.5 |
| Polyoxyethylene(8)cetylether | 0.2 |
| Octyldodecanol | 0.2 |
| Vitamin E acetate | 0.05 |
| Methylparaben | 0.2 |
| Talc | 0.9 |
| Pyrite | 0.05 |
| Ferric oxide | 0.02 |
| Ferrous oxide | 0.01 |
| Perfume | as appropriate |

(continued)

| Ingredients | Weight ratio (%) |
|---|---|
| Purified water | rest |

Make-up lotion was prepared by a conventional method.
4* Nonpurified cation-modified tara gum (INCl name: Caesalpinia Spinosa Hydroxypropyltrimonium Chloride

TABLE 39. Composition 21. Foundation

| Ingredients | Weight ratio (%) |
|---|---|
| Present invention (Sample 4) | 0.2 |
| Polyquaternium-7 | 0.1 |
| Polyquaternium-39 | 0.1 |
| Methylphenylpolysiloxane | 18.0 |
| Dimethylpolysiloxane | 7.0 |
| Squalane | 2.0 |
| Cetylisooctanoate | 2.0 |
| Organic modified mentonite | 1.8 |
| Diglycerildiisostearate | 1.6 |
| 1,3-butanediol | 4.0 |
| Silicone-treated talc | 10.0 |
| Silicone treated sericite | 12.0 |
| Silicone-treated titanium oxide | 8.0 |
| Silicone-treated pyrite | 2.2 |
| Silicone-treated ferric oxide | 0.7 |
| Monoglyceride stearate | 1.0 |
| Methylparaben | 0.2 |
| Perfume | as appropriate |
| Purified water | rest |

Foundation was prepared by a conventional method.

TABLE 40. Composition 22. Bath preparation

| Ingredients | Weight ratio (%) |
|---|---|
| Present invention (Sample 7) | 0.15 |
| Nonpurified cation-modified locust-bean gum (*5) | 0.15 |
| Magnesium palmitate | 2.0 |
| Calcium stearate | 3.0 |
| Liquid paraffin | 9.0 |
| Cetanol | 2.0 |
| Carboxyvinyl polymer | 0.15 |
| Triethanolamine | 0.13 |
| Squalane | 2.0 |

(continued)

| Ingredients | Weight ratio (%) |
|---|---|
| Perfume | as appropriate |
| Paraoxybenzoic acid methyl | 0.2 |
| Distilled water | rest |

Bath preparation was prepared by a conventional method.
*5) Nonpurified cation-modified locustbean gum (INCl name: Locust Bean Hydroxypropyltrimonium Chloride)

**Claims**

1. A cation-modified purified galactomannan polysaccharide prepared by partially substituting hydroxyl groups of a purified galactomannan polysaccharide with a quaternary nitrogen-containing group of Formula (1), the purified galactomannan polysaccharide being prepared by purified a crude galactomannan polysaccharide, comprising a main chain composed of mannose units and a side chain composed of galactose units, and containing 80 wt% of galactomannan:

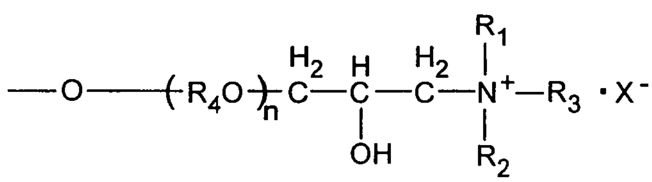

**(1)**

(where $R_1$ and $R_2$ each independently represent an alkyl group having 1 to 3 carbon atoms; $R_3$ represents an alkyl group having 1 to 24 carbon atoms; X-represents an anion; n is either 0 or 1 to 30; and, when n is 1 to 30, $(R_4O)_n$ represents a polymer residue of an alkylene oxide having 2 to 4 carbon atoms and forms a polyalkylene glycol chain composed of a single alkylene oxide and/or a polyalkylene glycol chain composed of two or more types of alkylene oxide), wherein the cation charge density derived from the quaternary nitrogen-containing group is in the range of 0.1 to 3.0 meq/g.

2. The cation-modified purified galactomannan polysaccharide according to claim 1, wherein an aqueous solution of the purified galactomannan polysaccharide containing 80 wt% or more of galactomannan forms a gel with an aqueous solution of xanthan gum and/or carrageenan.

3. The cation-modified purified galactomannan polysaccharide according to claim 1 or 2, wherein the purified galactomannan polysaccharide containing 80 wt% or more of galactomannan comprises amino chain composed of mannose and a side chain composed of galactose units and has a mannose to galactose ratio of 4:1.

4. The cation-modified galactomannan purified polysaccharide according to one of claims 1 to 3, wherein the purified galactomannan polysaccharide having a mannose to galactose ratio of 4:1 is a natural water-soluble gum obtained from albumens of seeds of locust bean *(Ceratonia Siliqua),* which is a perennial leguminous plant.

5. The cation-modified purified galactomannan polysaccharide according to claim 1 or 2, wherein the purified galactomannan polysaccharide containing 80 wt% or more of galactomannan comprises amino chain composed of mannose and a side chain composed of galactose units and has a mannose to galactose ratio of 3:1.

6. The cation-modified purified galactomannan polysaccharide according to claim 1, 2, or 5, wherein the purified galactomannan polysaccharide has a mannose to galactose ratio of 3:1 and is a natural water-soluble gum obtained from albumens of seeds of tara *(Caesalpinia Spinosa),* a leguminous plant.

7. The cation-modified purified galactomannan polysaccharide according to any one of claims 1 to 6, wherein the substitution of the hydroxyl groups in the purified galactomannan polysaccharide with the quaternary nitrogen-containing group is carried out through a reaction between galactomannan polysaccharide and one of a glycidyltrialkyl ammonium salt and a 3-halogeno-2-hydroxy-propyltrialkyl ammonium salt.

8. The cation-modified purified galactomannan polysaccharide according to any one of claims 1 to 7, wherein cation-modification of the purified galactomannan polysaccharide is conducted by adding an alkylene oxide having 2 to 4 carbon atoms to part of hydroxyl groups in the purified galactomannan polysaccharide, followed by substitution with a glycidyltrialkyl ammonium salt or a 3-halogeno-2-hydroxypropyltrialkyl ammonium salt functioning as a cationic modifier.

9. A cosmetic composition comprising the cation-modified galactomannan purified polysaccharide according to one of claims 1 to 8.

10. The cosmetic composition according to claim 9, wherein the content of the cation-modified purified galactomannan polysaccharide according to one of claims 1 to 8 is 0.05 to 5 wt% relative to 100 wt% of the entire composition.

11. The cosmetic composition according to claim 9 or 10, further comprising less than 5 wt% of a cationic water-soluble polymer and/or an amphoteric water-soluble polymer relative to 100 wt/% of the entire composition.

12. The cosmetic composition according to one of claims 9 to 11, further comprising an amideamine compound, a neutralizing agent such as an organic acid and/or an inorganic acid, a higher fatty acid, and/or a higher alcohol.

13. The cosmetic composition according to one of claims 9 to 12, further comprising silicone.

14. A hair treatment composition comprising the cosmetic composition according to one of claims 9 to 13.

**INTERNATIONAL SEARCH REPORT**

International application No.
PCT/JP2005/000995

A. CLASSIFICATION OF SUBJECT MATTER
Int.Cl$^7$ C08B37/00, A61K7/00, 7/06

According to International Patent Classification (IPC) or to both national classification and IPC

B. FIELDS SEARCHED

Minimum documentation searched (classification system followed by classification symbols)
Int.Cl$^7$ C08B37/00, A61K7/00, 7/06

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2005 |
| Kokai Jitsuyo Shinan Koho | 1971-2005 | Toroku Jitsuyo Shinan Koho | 1994-2005 |

Electronic data base consulted during the international search (name of data base and, where practicable, search terms used)
WPI(DIALOG), JICST FILE(JOIS)

C. DOCUMENTS CONSIDERED TO BE RELEVANT

| Category* | Citation of document, with indication, where appropriate, of the relevant passages | Relevant to claim No. |
|---|---|---|
| X<br><br>Y | JP 10-36403 A (Taiyo Kagaku Co., Ltd.),<br>10 February, 1998 (10.02.98),<br>Full text<br>(Family: none) | 1-7,9-11,13, 14<br>1-14 |
| Y | JP 2003-327603 A (Toho Chemical Industry Co., Ltd.),<br>19 November, 2003 (19.11.03),<br>Full text<br>(Family: none) | 1-14 |
| Y | JP 07-17825 A (Nichiden Kagaku Kabushiki Kaisha),<br>20 January, 1995 (20.01.95),<br>Full text<br>(Family: none) | 1-14 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

Date of the actual completion of the international search
14 April, 2005 (14.04.05)

Date of mailing of the international search report
10 May, 2005 (10.05.05)

Name and mailing address of the ISA/
Japanese Patent Office

Authorized officer

Facsimile No.

Telephone No.

Form PCT/ISA/210 (second sheet) (January 2004)

**INTERNATIONAL SEARCH REPORT**

International application No.
PCT/JP2005/000995

C (Continuation). DOCUMENTS CONSIDERED TO BE RELEVANT

| Category* | Citation of document, with indication, where appropriate, of the relevant passages | Relevant to claim No. |
|---|---|---|
| Y | JP 2000-103724 A (Toho Chemical Industry Co., Ltd.), 11 April, 2000 (11.04.00), Full text (Family: none) | 1-14 |
| Y | WO 1998/018828 A1 (RHONE-POULENC INC.), 07 May, 1998 (07.05.98), Full text; particularly, pages 1, 14 & EP 934343 A1 | 1-14 |
| Y | JP 05-239106 A (Mitsubishi Rayon Co., Ltd.), 17 September, 1993 (17.09.93), Full text (Family: none) | 1-14 |
| A | JP 11-507406 A (CIBUS PHARMACEUTICAL, INC.), 29 June, 1999 (29.06.99), Full text & WO 1996/040163 A1 | 1-14 |

Form PCT/ISA/210 (continuation of second sheet) (January 2004)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 47020635 A
- JP 4364111 A
- JP 7173029 A
- JP 3349219 B
- JP 2000103724 A
- JP 2003327603 A

### Non-patent literature cited in the description

- *FRAGRANCE JOURNAL,* 2003, vol. 10, 34